# EUROPÄISCHE PATENTSCHRIFT

(11) **EP 1 716 209 B1**
(45) Veröffentlichungstag und Bekanntmachung des Hinweises auf die Patenterteilung: **25.03.2009**
(21) Anmeldenummer: 04803283.3
(22) Anmeldetag: 26.11.2004
(51) Int. Cl.: A61K 8/49, A61Q 5/06

(54) **VERWENDUNG EINES INDOLYLTHIAZOLIUMAZOFARBSTOFFE ENTHALTENDES MITTEL ZUR FÄRBUNG VON KERATINFASERN**
USE OF COMPOSITION CONTAINING INDOLYL THIAZOLIUM AZO DYES FOR DYING KERATIN FIBERS
UTILISATION D'UNE COMPOSITION CONTENANT DES COLORANTS AZOÏQUES D'INDOLYLTHIAZOLIUM POUR COLORER LES FIBRES KÉRATINIQUES

(30) Priorität: 21.02.2004 DE 102004008607
(43) Veröffentlichungstag der Anmeldung: 02.11.2006
(73) Patentinhaber: Wella Aktiengesellschaft, 64274 Darmstadt (DE)
(72) Erfinder: PASQUIER, Cécile, CH-1723 Marly (CH); BUCLIN, Véronique, CH-1638 Morlon (CH); DUC-REICHLIN, Nadia, CH-1470 Lully (CH); KIENER, Caroline, CH-1731 Ependes (CH); BRAUN, Hans-Jürgen, CH-3182 Überstorf (CH)
(74) Vertreter: Bockhorst, Matthias
(86) Internationale Anmeldenummer: PCT/EP2004/013401
(87) Internationale Veröffentlichungsnummer: WO 2005/079734

(56) Entgegenhaltungen:
- BE-A1- 768 389
- CH-A- 567 074
- DE-A1- 2 114 747
- US-A- 4 104 268

## Beschreibung

Gegenstand der vorliegenden Erfindung ist die Verwendung eines Indolylthiazoliumazofarbstoffe enthaltenden Mittels zur Färbung von Keratinfasern, beispielsweise Wolle, Pelze und Haare.

Für die farbverändernde Behandlung von Keratinfasern werden in der Regel zwei Färbeverfahren angewendet. Im ersten Verfahren wird die Färbung mit sogenannten oxidativen oder permanenten Färbemitteln unter Verwendung einer Mischung aus verschiedenen Entwicklersubstanzen und Kupplersubstanzen und eines Oxidationsmittels erzeugt. Bei Bedarf können bei diesem Verfahren zur Abrundung des Färbeergebnisses oder zur Erzeugung von besonderen Farbeffekten sogenannte direktziehende (nicht-oxidative) Farbstoffe zugesetzt werden. Das zweite Verfahren bedient sich ausschließlich direktziehender Farbstoffe, die in einer geeigneten Trägermasse auf die Fasern aufgebracht werden. Dieses Verfahren ist einfach anzuwenden, ausgesprochen schonend und zeichnet sich durch eine geringe Schädigung der Keratinfaser aus. An die hierbei verwendeten direktziehenden Farbstoffe werden eine Vielzahl von Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Außerdem wird für die erzielten Färbungen eine gute Lichtechtheit, Säureechtheit und Reibechtheit gefordert. Die Vorteile von direktziehenden gegenüber oxidativen Färbungen liegen in der im allgemeinen geringeren Haarschädigung, da normalerweise mit niedrigeren pH-Werten (kleiner 9) und ohne Oxidationsmittel gearbeitet wird. Verschiedentlich werden Direktzieher auch als Nuancierhilfen in oxidativen Färbemitteln eingesetzt. Für ein direktziehendes (nicht-oxidatives) Färbemittel für Keratinfasern wird in der Regel eine Kombination von verschiedenen nicht-oxidativen Farbstoffen benötigt. Da die Auswahl an in Färbemitteln für Keratinfasern einsetzbaren Farbstoffen beschränkt ist, besteht weiterhin ein Bedarf an derartigen Farbstoffen.

Im Stand der Technik CH 567074 A; US 4104268 A, DE 2114747 A1 und BE 768389 werden kationische Azofarbstoffe beschrieben, welche auch Indolylthiazoliumazofarbstoffe umfassen. Die Verwendung derartiger Farbstoffe zur Färbung von Keratinfasern wird im Stand der Technik jedoch nicht beschrieben.

Es wurde nun gefunden, dass bestimmte Indolylthiazoliumazofarbstoffe Keratinfasern intensiv rot bis violett färben und eine besonders gute Lichtechtheit und Schweißbeständigkeit aufweisen.

Gegenstand der vorliegenden Erfindung ist daher die Verwendung eines mindestens einen Indolylthiazoliumazofarbstoff der allgemeinen Formel (I) enthaltenden Mittels zur Färbung von Keratinfasern, insbesonders menschlichen Haaren, 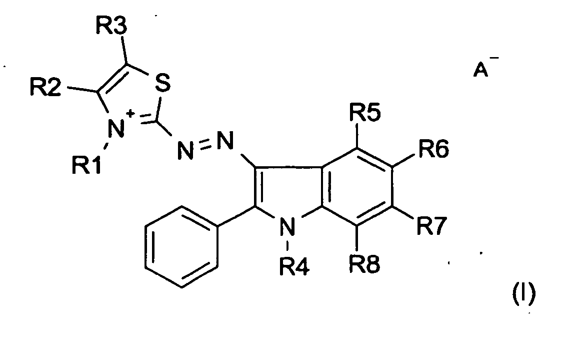 wobei
**R1** eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine (C₁-C₆)Alkoxy-(C₁-C₁₂)-alkylgruppe, eine Amino-(C₁-C₁₂)alkylgruppe, eine (C₁-C₆)-Alkylamino-(C₁-C₁₂)-alkylgruppe, eine Di-(C₁-C₆)-alkylamino-(C₁-C₁₂)alkylgruppe, eine Cyano-(C₁-C₁₂)-alkylgruppe, eine substituierte oder unsubstituierte Phenylgruppe oder eine substituierte oder unsubstituierte Benzylgruppe darstellt,
**R2** und **R3** gleich oder verschieden sein können und unabhängig voneinander Wasserstoff, ein Halogenatom (F, Cl, Br, J), eine gesättigte oder ungesättigte (C₁-C₁₂)-Alkylgruppe, eine (C₁-C₁₂)-Alkoxygruppe, eine Nitrogruppe, eine Aminogruppe, eine (C₁-C₁₂)-Alkylaminogruppe, eine Di(C₁-C₁₂)-alkylaminogruppe, eine (C₁-C₁₂)-Hydroxyalkylaminogruppe, eine Di(C₁-C₁₂)-hydroxyalkylaminogruppe, eine substituierte oder unsubstituierte Phenylgruppe oder eine substituierte oder unsubstituierte Heteroarylgruppe darstellen;
**R4** Wasserstoff, eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkylgruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe, eine substituierte oder unsubstituierte Benzylgruppe oder eine substituierte oder unsub-stituierte Heteroarylgruppe darstellt;
**R5, R6, R7,** und **R8** gleich oder verschieden sein können und unabhängig voneinander Wasserstoff, ein Halogenatom (F, Cl, Br, J), eine gesättigte oder ungesättigte (C₁-C₁₂)-Alkylgruppe, eine Hydroxygruppe, eine (C₁-C₁₂)-Alkoxygruppe, eine Nitrogruppe, eine Aminogruppe, eine (C₁-C₁₂)-Alkylaminogruppe oder eine Di(C₁-C₁₂)-alkylaminogruppe darstellen; und
**A⁻** gleich einem Anion einer organischen oder anorganischen Säure ist.

Unter den vorgenannten Verbindungen der Formel (I) sind solche bevorzugt, bei denen **R1** gleich einer gesättigten oder ungesättigten (C₁-C₁₂)-Alkylgruppe ist und **R4** gleich Wasserstoff, einer gesättigten oder ungesättigten (C₁-C₁₂)-Alkylgruppe, einer substituierten oder unsubstituierten Phenylgruppe ist. Besonders bevorzugt sind Verbindungen der Formel (I) bei denen **R1** gleich einer gesättigten (C₁-C₁₂)-Alkylgruppe und **R4** gleich Wasserstoff, einer gesättigten (C₁-C₁₂)-Alkylgruppe, oder einer unsubstituierten Phenylgruppe ist.
**A⁻** ist vorzugsweise gleich Chlorid, Bromid, Jodid, Hydrogensulfat, Sulfat, Toluolsulfonat, Benzolsulfonat, Monomethylsulfat, Hexafluorphosphat, Hexafluorantimonat, Tetrafluorborat, Tetraphenylborat, Formiat, Acetat oder Propionat, wobei das Chloridion, das Bromidion und das Monomethylsulfation besonders bevorzugt sind.

Als geeignete Verbindung der allgemeinen Formel (I) können beispielsweise genannt werden:
3-Methyl-2-[(1-methyl-2-phenyl-1H-indol-3-yl)azo]-thiazolium-chlorid,
3-Methyl-2-[(1-methyl-2-phenyl-1H-indol-3-yl)azo]-thiazolium-bromid,
3-Methyl-2-[(1-methyl-2-phenyl-1H-indol-3-yl)azo]-thiazolium- monomethylsulfat, 3,4-Dimethyl-2-[(1-methyl-2-phenyl-1H-indol-3-yl)azo]-thiazolium-chlorid, 3,4-Dimethyl-2-[(1-methyl-2-phenyl-1H-indol-3-yl)azo]-thiazolium-bromid, 3,4-Dimethyl-2-[(1-methyl-2-phenyl-1H-indol-3-yl)azo]-thiazolium-monomethylsulfat, 3,5-Dimethyl-2-[(1-methyl-2-phenyl-1 H-indol-3-yl)azo]-thiazolium-chlorid, 3,5-Dimethyl-2-[(1-methyl-2-phenyl-1H-indol-3-yl)azo]-thiazolium-bromid, 3,5-Dimethyl-2-[(1-methyl-2-phenyl-1 H-indol-3-yl)azo]-thiazolium-monomethylsulfat, 3,4,5-Trimethyl-2-[(1-methyl-2-phenyl-1H-indol-3-yl)azo]-thiazolium-chlorid, 3,4,5-Trimethyl-2-[(1-methyl-2-phenyl-1 H-indol-3-yl)azo]-thiazolium-bromid,
3,4,5-Trimethyl-2-[(1-methyl-2-phenyl-1H-indol-3-yl)azo]-thiazolium-monomethylsulfat, 5-Brom-3-methyl-2-[(1-methyl-2-phenyl-1 H-indol-3-yl)azo]-thiazolium-chlorid, 5-Brom-3-methyl-2-[(1-methyl-2-phenyl-1H-indol-3-yl)azo]-thiazolium-bromid, 5-Brom-3-methyl-2-[(1-methyl-2-phenyl-1 H-indol-3-yl)azo]-thiazolium- monomethylsulfat, 5-Methoxy-3-methyl-2-[(1-methyl-2-phenyl-1H-indol-3-yl)azo]-thiazolium-chlorid, 5-Methoxy-3-methyl-2-[(1-methyl-2-phenyl-1 H-indol-3-yl)azo]-thiazolium-bromid,
5-Methoxy-3-methyl-2-[(1-methyl-2-phenyl-1 H-indol-3-yl)azo]-thiazolium-monomethylsulfat, 5-Diethylamino-3-methyl-2-[(1 -methyl-2-phenyl-1 H-indol-3-yl)azo]-thiazolium-chlorid, 5-Diethylamino-3-methyl-2-[(1-methyl-2-phenyl-1 H-indol-3-yl)azo]-thiazolium-bromid, 5-Diethylamino-3-methyl-2-[(1-methyl-2-phenyl-1H-indol-3-yl)azo]-thiazolium-monomethylsulfat,
3-Methyl-2-[(1-methyl-2-phenyl-1H-indol-3-yl)azo]-5-nitro-thiazolium-chlorid, 3-Methyl-2-[(1-methyl-2-phenyl-1H-indol-3-yl)azo]-5-nitrothiazolium-bromid, 3-Methyl-2-[(1-methyl-2-phenyl-1H-indol-3-yl)azo]-5-nitro-thiazolium-monomethylsulfat, 3-Methyl-2-[(2-phenyl-1 H-indol-3-yl)azo]-thiazolium-chlorid, 3-Methyl-2-[(2-phenyl-1H-indol-3-yl)azo]-thiazolium-bromid, 3-Methyl-2-[(2-phenyl-1H-indol-3-yl)azo]-thiazolium-monomethylsulfat, 3,4-Dimethyl-2-[(2-phenyl-1 H-indol-3-yl)azo]-thiazolium-chlorid, 3,4-Dimethyl-2-[(2-phenyl-1 H-indol-3-yl)azo]-thiazolium-bromid, 3,4-Dimethyl-2-[(2-phenyl-1 H-indol-3-yl)azo]-thiazolium-monomethylsulfat, 3,5-Dimethyl-2-[(2-phenyl-1H-indol-3-yl)azo]-thiazolium-chlorid, 3,5-Dimethyl-2-[(2-phenyl-1 H-indol-3-yl)azo]-thiazolium-bromid, 3,5-Dimethyl-2-[(2-phenyl-1H-indol-3-yl)azo]-thiazolium-monomethylsulfat, 3,4,5-Trimethyl-2-[(2-phenyl-1 H-indol-3-yl)azo]-thiazolium-chlorid, 3,4,5-Trimethyl-2-[(2-phenyl-1H-indol-3-yl)azo]-thiazolium-bromid, 3,4,5-Trimethyl-2-[(2-phenyl-1H-indol-3-yl)azo]-thiazolium-monomethylsulfat, 5-Brom-3-methyl-2-[(2-phenyl-1 H-indol-3-yl)azo]-thiazolium-chlorid, 5-Brom-3-methyl-2-[(2-phenyl-1H-indol-3-yl)azo]-thiazolium-bromid, 5-Brom-3-methyl-2-[(2-phenyl-1H-indol-3-yl)azo]-thiazolium- monomethylsulfat, 5-Methoxy-3-methyl-2-[(2-phenyl-1H-indol-3-yl)azo]-thiazolium-chlorid, 5-Methoxy-3-methyl-2-[(2-phenyl-1H-indol-3-yl)azo]-thiazolium-bromid, 5-Methoxy-3-methyl-2-[(2-phenyl-1 H-indol-3-yl)azo]-thiazolium-monomethylsulfat, 5-Diethylamino-3-methyl-2-[(2-phenyl-1H-indol-3-yl)azo]-thiazolium-chlorid, 5-Diethylamino-3-methyl-2-[(2-phenyl-1H-indol-3-yl)azo]-thiazolium-bromid, 5-Diethylamino-3-methyl-2-[(2-phenyl-1 H-indol-3-yl)azo]-thiazolium- monomethylsulfat,
3-Methyl-5-nitro-2-[(2-phenyl-1 H-indol-3-yl)azo]-thiazolium-chlorid,
3-Methyl-5-nitro-2-[(2-phenyl-1 H-indol-3-yl)azo]-thiazolium-bromid,
3-Methyl-5-nitro-2-[(2-phenyl-1 H-indol-3-yl)azo]-thiazolium-monomethylsulfat, 2-[(1-Ethyl-2-phenyl-1 H-indol-3-yl)azo]-3-methyl-thiazolium-chlorid,

2-[(1-Ethyl-2-phenyl-1 H-indol-3-yl)azo]-3-methyl-thiazolium-bromid,
2-[(1-Ethyl-2-phenyl-1H-indol-3-yl)azo]-3-methyl-thiazolium-monomethylsulfat, 2-[(1-Ethyl-2-phenyl-1H-indol-3-yl)azo]-3,4-dimethyl-thiazolium-chlorid, 2-[(1-Ethyl-2-phenyl-1H-indol-3-yl)azo]-3,4-dimethyl-thiazolium-bromid, 2-[(1-Ethyl-2-phenyl-1H-indol-3-yl)azo]-3,4-dimethyl-thiazolium-monomethylsulfat, 2-[(1-Ethyl-2-phenyl-1H-indol-3-yl)azo]-3,5-dimethyl-thiazolium-chlorid, 2-[(1-Ethyl-2-phenyl-1 H-indol-3-yl)azo]-3,5-dimethyl-thiazolium-bromid, 2-[(1-Ethyl-2-phenyl-1H-indol-3-yl)azo]-3,5-dimethyl-thiazolium-monomethylsulfat, 2-[(1-Ethyl-2-phenyl-1H-indol-3-yl)azo]-3,4,5-trimethyl-thiazolium-chlorid, 2-[(1-Ethyl-2-phenyl-1H-indol-3-yl)azo]-3,4,5-trimethyl-thiazolium-bromid, 2-[(1-Ethyl-2-phenyl-1 H-indol-3-yl)azo]-3,4,5-trimethyl-thiazolium-monomethylsulfat, 5-Brom-2-[(1-ethyl-2-phenyl-1 H-indol-3-yl)azo]-3-methyl-thiazolium-chlorid, 5-Brom-2-[(1-ethyl-2-phenyl-1 H-indol-3-yl)azo]-3-methyl-thiazolium-bromid, 5-Brom-2-[(1-ethyl-2-phenyl-1 H-indol-3-yl)azo]-3-methyl-thiazolium-monomethylsulfat,
2-[(1-Ethyl-2-phenyl-1H-indol-3-yl)azo]-5-methoxy-3-methyl-thiazolium-chlorid, 2-[(1-Ethyl-2-phenyl-1H-indol-3-yl)azo]-5-methoxy-3-methyl-thiazolium-bromid, 2-[(1-Ethyl-2-phenyl-1H-indol-3-yl)azo]-5-methoxy-3-methyl-thiazolium-monomethylsulfat, 5-Diethylamino-2-[(1-ethyl-2-phenyl-1 H-indol-3-yl)azo]-3-methyl-thiazolium-chlorid, 5-Diethylamino-2-[(1-ethyl-2-phenyl-1H-indol-3-yl)azo]-3-methyl-thiazolium-bromid, 5-Diethylamino-2-[(1-ethyl-2-phenyl-1 H-indol-3-yl)azo]-3-methyl-thiazolium-monomethylsulfat, 2-[(1-Ethyl-2-phenyl-1H-indol-3-yl)azo]-3-methyl-5-nitro-thiazolium-chlorid, 2-[(1-Ethyl-2-phenyl-1H-indol-3-yl)azo]-3-methyl-5-nitro-thiazolium-bromid, 2-[(1 -Ethyl-2-phenyl-1 H-indol-3-yl)azo]-3-methyl-5-nitro-thiazolium-monomethylsulfat, 3-Methyl-2-[(1,2-diphenyl-1H-indol-3-yl)azo]-thiazolium-chlorid, 3-Methyl-2-[(1,2-diphenyl-1 H-indol-3-yl)azo]-thiazolium-bromid, 3-Methyl-2-[(1,2-diphenyl-1H-indol-3-yl)azo]-thiazolium-monomethylsulfat, 3,4-Dimethyl-2-[(1 ,2-diphenyl-1 H-indol-3-yl)azo]-thiazolium-chlorid, 3,4-Dimethyl-2-[(1,2-diphenyl-1H-indol-3-yl)azo]-thiazolium-bromid, 3,4-Dimethyl-2-[(1,2-diphenyl-1H-indol-3-yl)azo]-thiazolium-monomethylsulfat, 3,5-Dimethyl-2-[(1,2-diphenyl-1H-indol-3-yl)azo]-thiazolium-chlorid, 3,5-Dimethyl-2-[(1,2-diphenyl-1H-indol-3-yl)azo]-thiazolium-bromid, 3,5-Dimethyl-2-[(1,2-diphenyl-1H-indol-3-yl)azo]-thiazolium-monomethylsulfat, 3,4,5-Trimethyl-2-[(1,2-diphenyl-1H-indol-3-yl)azo]-thiazolium-chlorid, 3,4,5-Trimethyl-2-[(1,2-diphenyl-1 H-indol-3-yl)azo]-thiazolium-bromid, 3,4,5-Trimethyl-2-[(1,2-diphenyl-1H-indol-3-yl)azo]-thiazolium-monomethylsulfat, 5-Brom-3-methyl-2-[(1,2-diphenyl-1H-indol-3-yl)azo]-thiazolium-chlorid, 5-Brom-3-methyl-2-[(1,2-diphenyl-1H-indol-3-yl)azol-thiazolium-bromid, 5-Brom-3-methyl-2-[(1,2-diphenyl-1 H-indol-3-yl)azo]-thiazolium-monomethylsulfat, 5-Methoxy-3-methyl-2-[(1,2-diphenyl-1H-indol-3-yl)azo]-thiazolium-chlorid, 5-Methoxy-3-methyl-2-[(1,2-diphenyl-1 H-indol-3-yl)azo]-thiazolium-bromid,
5-Methoxy-3-methyl-2-[(1,2-diphenyl-1H-indol-3-yl)azo]-thiazolium-monomethylsulfat, 5-Diethylamino-3-methyl-2-[(1,2-diphenyl-1H-indol-3-yl)azo]-thiazolium-chlorid, 5-Diethylamino-3-methyl-2-[(1,2-diphenyl-1 H-indol-3-yl)azo]-thiazolium-bromid, 5-Diethylamino-3-methyl-2-[(1,2-diphenyl-1 H-indol-3-yl)azo]-thiazolium-monomethylsulfat, 3-Methyl-5-nitro-2-[(1,2-diphenyl-1H-indol-3-yl)azo]-thiazolium-chlorid, 3-Methyl-5-nitro-2-[(1,2-diphenyl-1H-indol-3-yl)azo]-thiazolium-bromid, 3-Methyl-5-nitro-2-[(1,2-diphenyl-1H-indol-3-yl)azo]-thiazolium-monomethylsulfat,
3-Ethyl-2-[(1-ethyl-2-phenyl-1 H-indol-3-yl)azo]-thiazolium-chlorid, 3-Ethyl-2-[(1-ethyl-2-phenyl-1 H-indol-3-yl)azo]-thiazolium-bromid, 3-Ethyl-2-[(1-ethyl-2-phenyl-1 H-indol-3-yl)azo]-thiazolium-monomethylsulfat, 3-Ethyl-2-[(1-ethyl-2-phenyl-1 H-indol-3-yl)azo]-4-methyl-thiazolium-chlorid, 3-Ethyl-2-[(1-ethyl-2-phenyl-1H-indol-3-yl)azo]-4-methyl-thiazolium-bromid,
3-Ethyl-2-[(1-ethyl-2-phenyl-1H-indol-3-yl)azo]-4-methyl-thiazolium- monomethylsulfat, 3-Ethyl-2-[(1-ethyl-2-phenyl-1H-indol-3-yl)azo]-5-methyl-thiazolium-chlorid, 3-Ethyl-2-[(1-ethyl-2-phenyl-1H-indol-3-yl)azo]-5-methyl-thiazolium-bromid, 3-Ethyl-2-[(1-ethyl-2-phenyl-1H-indol-3-yl)azo]-5-methyl-thiazolium- monomethylsulfat, 3-Ethyl-2-[(1-ethyl-2-phenyl-1 H-indol-3-yl)azo]-4,5-dimethyl-thiazolium-chlorid, 3-Ethyl-2-[(1-ethyl-2-phenyl-1 H-indol-3-yl)azo]-4,5-dimethyl-thiazolium-bromid, 3-Ethyl-2-[(1-ethyl-2-phenyl-1H-indol-3-yl)azo]-4,5-dimethyl-thiazolium-monomethylsulfat, 5-Brom-3-ethyl-2-[(1-ethyl-2-phenyl-1H-indol-3-yl)azo]-thiazolium-chlorid, 5-Brom-3-ethyl-2-[(1-ethyl-2-phenyl-1H-indol-3-yl)azo]-thiazolium-bromid, 5-Brom-3-ethyl-2-[(1-ethyl-2-phenyl-1H-indol-3-yl)azo]-thiazolium-monomethylsulfat, 3-Ethyl-2-[(1-ethyl-2-phenyl-1 H-indol-3-yl)azo]-5-methoxy-thiazolium-chlorid, 3-Ethyl-2-[(1-ethyl-2-phenyl-1 H-indol-3-yl)azo]-5-methoxy-thiazolium-bromid, 3-Ethyl-2-[(1-ethyl-2-phenyl-1H-indol-3-yl)azo]-5-methoxy-thiazolium-monomethylsulfat, 3-Ethyl-5-diethylamino-2-[(1-ethyl-2-phenyl-1 H-indol-3-yl)azo]-thiazolium-chlorid,
3-Ethyl-5-diethylamino-2-[(1-ethyl-2-phenyl-1H-indol-3-yl)azo]-thiazolium-bromid, 3-Ethyl-5-diethylamino-2-[(1-ethyl-2-phenyl-1H-indol-3-yl)azo]-thiazolium-monomethylsulfat, 3-Ethyl-2-[(1-methyl-2-phenyl-1H-indol-3-yl)azo]-thiazolium-chlorid, 3-Ethyl-2-[(1-methyl-2-phenyl-1H-indol-3-yl)azo]-thiazolium-bromid, 3-Ethyl-2-[(1-methyl-2-phenyl-1 H-indol-3-yl)azo]-thiazolium-monomethylsulfat, 2-[(1-Methyl-2-phenyl-1H-indol-3-yl)azo]-3-propyl-thiazolium-chlorid, 2-[(1-Methyl-2-phenyl-1H-indol-3-yl)azo]-3-propyl-thiazolium-bromid, 2-[(1-Methyl-2-phenyl-1 H-indol-3-yl)azo]-3-propyl-thiazolium-monomethylsulfat, 3-Hydroxyethyl-2-[(1-methyl-2-phenyl-1 H-indol-3-yl)azo]-thiazolium-chlorid, 3-Hydroxyethyl-2-[(1-methyl-2-phenyl-1H-indol-3-yl)azo]-thiazolium-bromid, 3-Hydroxyethyl-2-[(1-methyl-2-phenyl-1H-indol-3-yl)azo]-thiazolium-monomethylsulfat,
2-[(1-Methyl-2-phenyl-1 H-indol-3-yl)azo]-3-(2-propenyl)-thiazolium-chlorid,
2-[(1-Methyl-2-phenyl-1 H-indol-3-yl)azo]-3-(2-propenyl)-thiazolium-bromid und 2-[(1-Methyl-2-phenyl-1H-indol-3-yl)azo]-3-(2-propenyl)-thiazolium-monomethylsulfat.

Besonders bevorzugte Verbindungen der Formel (I) sind 3-Methyl-2-[(1-methyl-2-phenyl-1 H-indol-3-yl)azo]-thiazolium-chlorid, 3-Methyl-2-[(1-methyl-2-phenyl-1 H-indol-3-yl)azo]-thiazolium-bromid, 3-Methyl-2-[(1-methyl-2-phenyl-1 H-indol-3-yl)azo]-thiazolium-monomethylsulfat, 3,4-Dimethyl-2-[(1-methyl-2-phenyl-1 H-indol-3-yl)azo]-thiazolium-chlorid, 3,4-Dimethyl-2-[(1-methyl-2-phenyl-1 H-indol-3-yl)azo]-thiazolium-bromid, 3,4-Dimethyl-2-[(1-methyl-2-phenyl-1H-indol-3-yl)azo]-thiazolium-monomethylsulfat, 3,5-Dimethyl-2-[(1-methyl-2-phenyl-1H-indol-3-yl)azo]-thiazolium-chlorid, 3,5-Dimethyl-2-[(1-methyl-2-phenyl-1 H-indol-3-yl)azo]-thiazolium-bromid, 3,5-Dimethyl-2-[(1 -methyl-2-phenyl-1 H-indol-3-yl)azo]-thiazolium-monomethylsulfat, 3,4,5-Trimethyl-2-[(1-methyl-2-phenyl-1H-indol-3-yl)azo]-thiazolium-chlorid, 3,4,5-Trimethyl-2-[(1-methyl-2-phenyl-1 H-indol-3-yl)azo]-thiazolium-bromid, 3,4,5-Trimethyl-2-[(1-methyl-2-phenyl-1 H-indol-3-yl)azo]-thiazolium-monomethylsulfat, 5-Brom-3-methyl-2-[(1-methyl-2-phenyl-1H-indol-3-yl)azo]-thiazolium-chlorid, 5-Brom-3-methyl-2-[(1-methyl-2-phenyl-1 H-indol-3-yl)azo]-thiazolium-bromid, 5-Brom-3-methyl-2-[(1-methyl-2-phenyl-1H-indol-3-yl)azo]-thiazolium-monomethylsulfat, 5-Methoxy-3-methyl-2-[(1-methyl-2-phenyl-1H-indol-3-yl)azo]-thiazolium-chlorid, 5-Methoxy-3-methyl-2-[(1-methyl-2-phenyl-1H-indol-3-yl)azo]-thiazolium-bromid, 5-Methoxy-3-methyl-2-[(1-methyl-2-phenyl-1 H-indol-3-yl)azo]-thiazolium-monomethylsulfat, 3-Methyl-2-[(2-phenyl-1 H-indol-3-yl)azo]-thiazolium-chlorid, 3-Methyl-2-[(2-phenyl-1 H-indol-3-yl)azo]-thiazolium-bromid, 3-Methyl-2-[(2-phenyl-1 H-indol-3-yl)azo]-thiazolium-monomethylsulfat, 3,4-Dimethyl-2-[(2-phenyl-1H-indol-3-yl)azo]-thiazolium-chlorid, 3,4-Dimethyl-2-[(2-phenyl-1 H-indol-3-yl)azo]-thiazolium-bromid, 3,4-Dimethyl-2-[(2-phenyl-1 H-indol-3-yl)azo]-thiazolium-monomethylsulfat, 3,5-Dimethyl-2-[(2-phenyl-1 H-indol-3-yl)azo]-thiazolium-chlorid, 3,5-Dimethyl-2-[(2-phenyl-1 H-indol-3-yl)azo]-thiazolium-bromid, 3,5-Dimethyl-2-[(2-phenyl-1 H-indol-3-yl)azo]-thiazolium-monomethylsulfat, 3,4,5-Trimethyl-2-[(2-phenyl-1H-indol-3-yl)azo]-thiazolium-chlorid, 3,4,5-Trimethyl-2-[(2-phenyl-1 H-indol-3-yl)azo]-thiazolium-bromid, 3,4,5-Trimethyl-2-[(2-phenyl-1 H-indol-3-yl)azo]-thiazolium-monomethylsulfat, 5-Brom-3-methyl-2-[(2-phenyl-1 H-indol-3-yl)azo]-thiazolium-chlorid, 5-Brom-3-methyl-2-[(2-phenyl-1 H-indol-3-yl)azo]-thiazolium-bromid, 5-Brom-3-methyl-2-[(2-phenyl-1 H-indol-3-yl)azo]-thiazolium-monomethylsulfat, 5-Methoxy-3-methyl-2-[(2-phenyl-1 H-indol-3-yl)azo]-thiazolium-chlorid, 5-Methoxy-3-methyl-2-[(2-phenyl-1H-indol-3-yl)azo]-thiazolium-bromid, 5-Methoxy-3-methyl-2-[(2-phenyl-1H-indol-3-yl)azo]-thiazolium-monomethylsulfat, 3-Methyl-2-[(1,2-diphenyl-1 H-indol-3-yl)azo]-thiazolium-chlorid, 3-Methyl-2-[(1,2-diphenyl-1 H-indol-3-yl)azo]-thiazolium-bromid, 3-Methyl-2-[(1,2-diphenyl-1 H-indol-3-yl)azo]-thiazolium-monomethylsulfat, 3,4-Dimethyl-2-[(1,2-diphenyl-1H-indol-3-yl)azo]-thiazolium-chlorid, 3,4-Dimethyl-2-[(1,2-diphenyl-1 H-indol-3-yl)azo]-thiazolium-bromid, 3,4-Dimethyl-2-[(1,2-diphenyl-1 H-indol-3-yl)azo]-thiazolium-monomethylsulfat, 3,5-Dimethyl-2-[(1,2-diphenyl-1 H-indol-3-yl)azo]-thiazolium-chlorid, 3,5-Dimethyl-2-[(1,2-diphenyl-1H-indol-3-yl)azo]-thiazolium-bromid, 3,5-Dimethyl-2-[(1,2-diphenyl-1 H-indol-3-yl)azo]-thiazolium-monomethylsulfat, 3,4,5-Trimethyl-2-[(1,2-diphenyl-1H-indol-3-yl)azo]-thiazolium-chlorid, 3,4,5-Trimethyl-2-[(1,2-diphenyl-1 H-indol-3-yl)azo]-thiazolium-bromid, 3, 4,5-Trimethyl-2-[(1,2-diphenyl-1 H-indol-3-yl)azo]-thiazolium-monomethylsulfat, 5-Brom-3-methyl-2-[(1,2-diphenyl-1 H-indol-3-yl)azo]-thiazolium-chlorid, 5-Brom-3-methyl-2-[(1,2-diphenyl-1 H-indol-3-yl)azo]-thiazolium-bromid, 5-Brom-3-methyl-2-[(1,2-diphenyl-1 H-indol-3-yl)azo]-thiazolium-monomethylsulfat, 5-Methoxy-3-methyl-2-[(1,2-diphenyl-1 H-indol-3-yl)azo]-thiazolium-chlorid, 5-Methoxy-3-methyl-2-[(1,2-diphenyl-1H-indol-3-yl)azo]-thiazolium-bromid und 5-Methoxy-3-methyl-2-[(1,2-diphenyl-1 H-indol-3-yl)azo]-thiazolium-monomethylsulfat.

Die Verbindungen der Formel (I) sind in dem Färbemittel der erfindungsgemäßen Verwendung vorzugsweise in einer Menge von 0,01 bis 10 Gewichtsprozent, insbesondere 0,1 bis 8 Gewichtsprozent, enthalten.

Das erfindungsgemäße Färbemittel enthält neben den Farbstoffen der Formel (I) zusätzlich noch weitere bekannte direktfärbende Farbstoffe aus der Gruppe bestehend aus Nitrofarbstoffen, Azofarbstoffen, Anthrachinonfarbstoffen und Triphenylmethanfarbstoffen, alleine oder im Gemisch miteinander, beispielsweise 1,4-Bis[(2-hydroxyethyl)-amino]-2-nitrobenzol, 1-(2-Hydroxyethyl)amino-2-nitro-4-[di(2-hydroxyethyl)amino]-benzol, (HC Blue No. 2), 1-Amino-3-methyl-4-[(2-hydroxyethyl)amino]-6-nitrobenzol, (HC Violet No. 1), 4-[Ethyl-(2-hydroxyethyl)-amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol, (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol, (HC Violet No. 2), 1-Methylamino-4-[methyl-(2,3-dihydroxypropyl)amino]-2-nitrobenzol (HC Blue No. 6), 2-((4-Amino-2-nitrophenyl)-amino)-5-dimethylamino-benzoesäure (HC Blue No. 13), 1-(2-Amino-ethylamino)-4-[di(2-hydroxyethyl)amino]-2-nitrobenzol, 4-(Di(2-hydroxyethyl)amino)-2-nitro-1-phenylamino-benzol, 1-Amino-4-[(2-hydroxyethyl)amino]-2-nitrobenzol (HC Red No. 7), 2-Amino-4,6-dinitrophenol, 1,4-Diamino-2-nitrobenzol (CI76070), 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-((2-Hydroxyethyl)methylamino)-1-(methyl-amino)-2-nitrobenzol, 1-Amino-4-((2,3-dihydroxypropyl)amino)-5-methyl-2-nitrobenzol, 1-Amino-4-(methylamino)-2-nitrobenzol, 4-Amino-2-nitro-1-((prop-2-en-1-yl)amino)-benzol, 4-Amino-3-nitrophenol, 4-[(2-Hydroxyethyl)amino]-3-nitrophenol, 4-[(2-Nitrophenyl)amino]phenol (HC Orange No. 1), 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol, (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 6-Amino-3-((2-hydroxyethyl)-amino)-2-nitropyridin, 3-Amino-6-((2-hydroxyethyl)amino)-2-nitropyridin, 3-Amino-6-(ethylamino)-2-nitropyridin, 3-((2-Hydroxyethyl)amino)-6-(methylamino)-2-nitropyridin, 3-Amino-6-(methylamino)-2-nitropyridin, 6-(Ethylamino)-3-((2-hydroxyethyl)amino)-2-nitropyridin, 1,2,3,4-Tetrahydro-6-nitrochinoxalin, 7-Amino-3,4-dihydro-6-nitro-2H-1,4-benzoxazin (HC Red No. 14), 1,2-Diamino-4-nitrobenzol (CI76020), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol, (HC Yellow No. 4), 1-[(2-Hydroxyethyl)amino]-2-nitrobenzol (HC Yellow No. 2), 2-(Di(2-hydroxyethyl)amino)-5-nitrophenol, 2-[(2-Hydroxyethyl)amino]-1-methoxy-5-nitrobenzol, 2-Amino-3-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid, (HC Yellow No.9), 1-[(2-Ureidoethyl)amino]-4-nitrobenzol, 4-[(2,3-Dihydroxypropyl)amino]-3-nitro-1-trifluormethyl-benzol, (HC Yellow No. 6), 1-Chlor-2,4-bis[(2-hydroxyethyl)amino]-5-nitrobenzol (HC Yellow No. 10), 1-Amino-4-((2-aminoethyl)amino)-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol, (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 3-((2-Hydroxyethyl)amino)-4-methyl-1-nitrobenzol, 4-Chlor-3-((2-hydroxyethyl)amino)-1-nitrobenzol, 2,4-Dinitro-1-hydroxy-naphthalin. 1,4-Di[(2,3-dihydroxypropyl)amino]-9,10-anthrachinon, 1,4-Di[(2-hydroxyethyl)amino]-9,10-anthrachinon (CI61545, Disperse Blue 23), 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Amino-4-hydroxy-9,10-anthrachinon (CI60710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (CI75470, Natural Red 4), 1-[(3-Aminopropyl)amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1,4-Diamino-9,10-anthrachinon (CI61100, Disperse Violet No. 1), 1-Amino-4-(methylamino)-9,10-anthrachinon (CI61105, Disperse Violet No. 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-(6-((3-Chlor-4-(methylamino)phenyl)imino)-4-methyl-3-oxo-1,4-cyclohexadien-1-yl)harnstoff (HC Red No. 9), 2-((4-(Di(2-hydroxyethyl)-amino)phenyl)amino)-5-((2-hydroxyethyl)amino)-2,5-cyclohexadien-1,4-dion (HC Green No. 1), 5-Hydroxy-1,4-naphthochinon (CI75500, Natural Brown No. 7), 2-Hydroxy-1,4-naphthochinon (CI75480, Natural Orange No. 6), 1,2-Dihydro-2-(1,3-dihydro-3-oxo-2H-indol-2-yliden)-3H-indol-3-on (CI73000), 1,3-Bis(dicyanomethylen)indan, 9-(Dimethylamino)-benzo[a]-phenoxazin-7-ium-chlorid (CI51175; Basic Blue No. 6), Di[4-(diethylamino)phenyl][4-(ethylamino)naphthyl]carbenium-chlorid (CI42595; Basic Blue No. 7), Di-(4-(dimethylamino)phenyl)-(4-(methyl-phenylamino)-naphthalin-1-yl)carbenium-chlorid (CI42563; Basic Blue No. 8), 3,7-Di(dimethylamino)phenothiazin-5-ium-chlorid (CI52015; Basic Blue No. 9), Di[4-(dimethylamino)phenyl][4-(phenylamino)naphthyl]carbeniumchlorid (CI44045; Basic Blue No. 26), 2-[(4-(Ethyl(2-hydroxyethyl)-amino)phenyl)azo]-6-methoxy-3-methyl-benzothiazolium-methylsulfat (CI11154; Basic Blue No. 41), Basic Blue No. 77, 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl][4-(methylamino)phenyl]carbenium-chlorid (CI42535; Basic Violet No. 1), Tri(4-amino-3-methylphenyl)carbenium-chlorid (CI42520; Basic Violet No. 2), Tris[4-(dimethylamino)phenyl]carbenium-chlorid (CI42555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (CI21010;Basic Brown No. 4), 1-[(4-Aminophenyl)azo]-7-(trimethylammonio)-2-naphtholchlorid (CI12250; Basic Brown No. 16), 3-[(4-Amino-2,5-dimethoxyphenyl)azo]-N,N,N-trimethylbenzolaminium-chlorid (CI112605, Basic Orange No. 69), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 2-((4-Aminophenyl)azo)-1,3-dimethyl-1H-imidazol-3-ium-chlorid (Basic Orange No. 31), 3,7-Diamino-2,8-dimethyl-5-phenylphenazinium-chlorid (CI50240; Basic Red No. 2), 1,4-Dimethyl-5-[(4-(dimethylamino)phenyl)-azo]-1,2,4-triazolium-chlorid (CI11055; Basic Red No. 22), 1,3-Dimethyl-2-((4-dimethylamino)phenyl)azo-1H-imidazol-3-ium-chlorid (Basic Red No. 51), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)-phenyl)azo]-pyrazol-5-on-chlorid (CI12719; Basic Yellow No. 57), Di[4-(dimethylamino)phenyl]iminomethan-hydrochlorid (CI41000; Basic Yellow No. 2), 1-Methyl-4-((methyl-phenylhydrazono)methyl)-pyridinium-methylsulfat (Basic Yellow No. 87), Bis[4-(diethylamino)phenyl]-phenylcarbenium-hydrogensulfat(1:1) (CI42040; Basic Green No. 1), Di(4-(dimethylamino)phenyl)-phenylmethanol (CI42000; Basic Green No. 4), 1-(2-Morpholiniumpropylamino)-4-hydroxy-9,10-anthrachinon-methylsulfat, 1-[(3-(Dimethyl-propylaminium)propyl)amino]-4-(methylamino)-9,10-anthrachinon-chlorid, 1-[Di(2-hydroxyethyl)amino]-3-methyl-4-[(4-nitrophenyl)azo]-benzol (CI11210, Disperse Red No. 17), 1-[Di(2-hydroxyethyl)amino]-4-[(4-nitrophenyl)azo]-benzol, (Disperse Black No. 9), 4-[(4-Aminophenyl)azo]-1-[di(2-hydroxyethyl)amino]-3-methylbenzol, (HC Yellow No. 7), 2,6-Diamino-3-[(pyridin-3-yl)azo]-pyridin, 2-((4-(Acetylamino)phenyl)azo)-4-methylphenol (CI11855; Disperse Yellow No. 3), 2-((4-(Ethyl(2-hydroxyethyl)amino)-2-methylphenyl)azo)-5-nitro-1,3-thiazol (CI111935; Disperse Blue No. 106), 6-Hydroxy-5-[(4-sulfophenyl)azo]-2-naphthalinsulfonsäure-Dinatriumsalz (CI15985; Food Yellow No. 3; FD&C Yellow No. 6), 2,4-Dinitro-1-naphthol-7-sulfonsäure-Dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (CI47005; D&C Yellow No. 10; Food Yellow No. 13, Acid Yellow No. 3), 5-Hydroxy-1-(4-sulfophenyl)-4-[(4-sulfophenyl)azo]pyrazol-3-carbonsäure-Trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 4-((4-Amino-3-sulfophenyl)azo)benzolsulfonsäure-Dinatriumsalz (CI13015, Acid Yellow No. 9), 5-[(2,4-Dinitrophenyl)-amino]-2-phenylamino-benzolsulfonsäure-Natriumsalz (CI10385; Acid Orange No. 3), 4-[(2,4-Dihydroxyphenyl)azo]-benzolsulfonsäure-Mononatriumsalz (CI14270; Acid Orange No. 6), 4-[(2-Hydroxynaphth-1-yl)azo]-benzolsulfonsäure-Natriumsalz (CI15510; Acid Orange No. 7), 4-((2-Hydroxy-naphthalin-1-yl)azo)-3-methyl-benzolsulfonsäure-Natriumsalz (CI15575; Acid Orange No. 8), 4-[(2,4-Dihydroxy-3-[(2,4-dimethylphenyl)azo]phenyl)azo]-benzolsulfonsäure-Natriumsalz (CI20170; Acid Orange No. 24), 3',6'-Dihydroxy-4',5'-diiodospiro-(isobenzofuran-1(3H)-9'-(9H)xanthen)-3-on (CI45425, D&C Orange No. 10), 4-Hydroxy-3-[(4-sulfonaphth-1-yl)azo]-1-naphthalin-sulfonsäure-Dinatriumsalz (CI14720; Acid Red No. 14), 4-Hydroxy-3-[(2-methoxyphenyl)azo]-1-naphthalinsulfonsäure-mononatriumsalz (CI14710; Acid Red No. 4), 6-Hydroxy-5-[(4-sulfonaphth-1-yl)azo]-2,4-naphthalindisulfonsäure-Trinatriumsalz (CI16255; Ponceau 4R; Acid Red No. 18), 3-Hydroxy-4-[(4-sulfonaphth-1-yl)azo]-2,7-naphthalin-disulfonsäure-Trinatriumsalz (CI16185; Acid Red No. 27), 8-Amino-1-hydroxy-2-(phenylazo)-3,6-naphthalin-disulfonsäure-Dinatriumsalz (CI17200; Acid Red No. 33), 5-(Acetylamino)-4-hydroxy-3-[(2-methylphenyl)azo]-2,7-naphthalin-disulfonsäure-Dinatriumsalz (CI18065; Acid Red No. 35), 2-(3-Hydroxy-2,4,5,7-tetrajodo-dibenzopyran-6-on-9-yl)-benzoesäure-Dinatriumsalz (CI45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)-phenyl)azo]-7-naphthol-1,3-disulfonsäure-Dinatriumsalz (CI27290; Acid Red No. 73), 2',4',5',7'-Tetrabrom-3',6'-dihydroxyspiro[isobenzofuran-1(3H),9'-[9H]-xanthen]-3-on-Dinatriumsalz (CI45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-Dinatriumsalz (CI45410; Acid Red No. 92), 3',6'-Dihydroxy-4',5'-diiodospiro[isobenzofuran-1(3H),9'(9H)-xanthen]-3-on-Dinatriumsalz (CI45425; Acid Red No. 95), 2-Hydroxy-3-((2-hydroxynaphth-1-yl)azo)-5-nitrobenzolsulfonsäure-Mononatriumsalz (CI15685; Acid Red No. 184), (2-Sulfophenyl)di[4-(ethyl((4-sulfophenyl)methyl)-amino)phenyl]-carbenium-Dinatriumsalz, Betain (CI42090; Acid Blue No. 9; FD&C Blue No. 1), 3-Hydroxy-4-((4-methyl-2-sulfophenyl)azo)-2-naphthalincarbonsäure-Dinatriumsalz (CI15850; D&C Red No. 6), 6-Hydroxy-5-((2-methoxy-5-methyl-4-sulfophenyl)azo)-2-naphthalinsulfonsäure-Dinatriumsalz (CI16035; FD&C Red 40), 1,4-Bis[(2-sulfo-4-methylphenyl)amino]-9,10-anthrachinon-Dinatriumsalz (CI 61570; Acid Green No. 25), Bis[4-(dimethylamino)phenyl]-(3,7-disulfo-2-hydroxynaphth-1-yl)-carbenium-inneres Salz, Mononatriumsalz (CI44090; Food Green No. 4; Acid Green No. 50), Bis[4-(diethylamino)phenyl](2,4-disulfophenyl)carbenium-inneres Salz, Natriumsalz (2:1) (CI42045; Food Blue No. 3; Acid Blue No. 1), Bis[4-(diethylamino)phenyl](5-hydroxy-2,4-disulfophenyl)carbenium-inneres Salz, Calciumsalz (2:1) (CI42051; Acid Blue No. 3), 1-Amino-4-(cyclohexylamino)-9,10-anthrachinon-2-sulfonsäure-Natriumsalz (CI62045; Acid Blue No. 62), 3,3-Bis(3,5-dibrom-4-hydroxyphenyl)-4,5,6,7-tetrabrom-2,1(3h)-benzoxathiol-1,1-dioxid, 1-Amino-4-(phenylamino)-9,10-anthrachinon-2-sulfonsäure (CI62055; Acid Blue No. 25), 2-(1,3-Dihydro-3-oxo-5-sulfo-2H-indol-2-yliden)-2,3-dihydro-3-oxo-1H-indol-5-sulfonsäure-Dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, Mononatriumsalz (CI45190; Acid Violet No. 9), 1-Hydroxy-4-[(4-methyl-2-sulfophenyl)amino]-9,10-anthrachinon-Natriumsalz (CI60730; D&C Violet No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäure-Dinatriumsalz (CI20470; Acid Black No. 1), 3-Hydroxy-4-[(2-hydroxynaphth-1-yl)azo]-7-nitro-1-naphthalin-sulfonsäure-Chromkomplex (3:2) (CI15711; Acid Black No. 52), 3-[(2,4-Dimethyl-5-sulfophenyl)azo]-4-hydroxy-1-naphthalinsulfonsäure-Dinatriumsalz (Cl14700; Food Red No. 1; Ponceau SX; FD&C Red No. 4), 4-(Acetylamino)-hydroxy-6-[(7-sulfo-4-[(4-sulfophenyl)-azo]naphth-1-yl)azo]-1,7-naphthalindisulfonsäure-Tetranatriumsalz (CI28440; Food Black No. 1) und 3-Hydroxy-4-(3-methyl-5-oxo-1-phenyl-4,5-dihydro-1H-pyrazol-4-ylazo)-naphthalin-1-sulfonsäure-Natriumsalz, Chrom-Komplex (Acid Red No. 195).

Die vorstehend genannten direktziehenden Farbstoffe können in einer Gesamtmenge von etwa 0,01 bis 4 Gewichtsprozent enthalten sein, wobei der Gesamtgehalt an Farbstoffen in dem erfindungsgemäßen Färbemittel vorzugsweise etwa 0,01 bis 10 Gewichtsprozent, insbesonders 0,1 bis 8 Gewichtsprozent, beträgt.

Oxidationsfarbstoffvorstufen (Entwicklersubstanzen und Kupplersubstanzen), wie zum Beispiel o,p,m-Phenylendiamine, o,p,m-Aminophenole, Diphenole oder 4,5-Diamino-pyrazole, sowie geeignete Oxidationsmittel (insbesondere Wasserstoffperoxid und dessen Adddukte) werden dem erfindungsgemäßen Färbemittel auch zugesetzt.

Diese Entwicklersubstanzen und Kuppiersubstanzen können in dem Färbemittel jeweils in einer Gesamtmenge von etwa 0,01 bis 20 Gewichtsprozent, vorzugsweise etwa 0,1 bis 10 Gewichtsprozent und insbesondere 0,1 bis 5 Gewichtsprozent, enthalten sein.

Die Zubereitungsform des erfindungsgemäßen Färbemittels kann beispielsweise eine Lösung, insbesondere eine wässrige oder wässrigalkoholische Lösung sein, eine Creme, ein Gel, eine tensidhaltige schäumende Lösung (Shampoo, Aerosol), eine Emulsion oder ein anderer für die Anwendung auf dem Haar geeigneter, wasserhaltiger Träger sein. Es ist ebenfalls möglich, dass das erfindungsgemäße Färbemittel in Form von Pellets, Granulaten oder Pulvern vorliegt, die vor der Anwendung in einer wässrigen Zubereitung -beispielsweise in Wasser oder einer wässrigen Oxidationsmittelzubereitung- gelöst werden.
Die Zusammensetzung dieser Mittel stellt eine Mischung der Farbstoffkomponente mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische einwertige oder mehrwertige Alkohole, deren Ester und Ether, beispielsweise Alkanole, insbesondere mit 1 bis 4C-Atomen, beispielsweise Ethanol, Propanol oder Isopropanol, Butanol, Isobutanol, zweiwertige und dreiwertige Alkohole, insbesondere solche mit 2 bis 6 C-Atomen, beispielweise Ethylenglykol, Propylenglykol, 1,3-Propandiol, 1,4-Butandiol, 1,5-Pentandiol, 1,6-Hexandiol, 1,2,6-Hexantriol, Glycerin, Diethylenglykol, Dipropylenglykol, Polyalkylenglykole, wie Triethylenglykol, Polyethylenglykol, Tripropylenglykol, Polypropylenglykol, niedere Alkylether von mehrwertigen Alkoholen, wie Ethylenglykolmonomethylether, Ethylenglykolmonoethylether, Ethylenglykolmonopropylether, Ethylenglykolmonobuthylether, Diethylenglykolmonomethylether, Diethylenglykolmonoethylether, Triethylenglykolmonomethylether oder Triethylenglykolmonoethylether, Ketone und Ketoalkohole, insbesondere solche mit 3 bis 7 C-Atomen, wie zum Beispiel Aceton, Methylethylketon, Diethylketon, Methylisobutylketon, Methylphenylketon, Cyclopentanon, Cyclohexanon, und Diacetonalkohol, Ether, wie zum Beispiel Dibuthylether, Tetrahydrofuran, Dioxan, Diisopropylether, Ester wie zum Beispiel Ethylformiat, Methylformiat, Methylacetat, Ethylacetat, Propylacetat, Butylacetat, Phenylacetat, Ethylenglykolmonoethyletheracetat, und Essigsäurehydroxyethylester, Amide wie zum Beispiel Dimethylformamid und Dimethylacetamid, N-Methylpyrrolidon, sowie Harnstoff, Tetramethylharnstoff und Thiodiglykol.

Weiterhin können in dem erfindungsgemäßen Färbemittel Netzmittel oder Emulgatoren aus den Klassen der anionischen, kationischen, amphoteren, nichtionogenen oder zwitterionischen oberflächenaktiven Substanzen wie zum Beispiel Fettalkoholsulfate, oxethylierte Fettalkoholsulfate, Alkylsulfonate, Alkylbenzolsulfonate, α-Olefinsulfonate, Alkyltrimethylammoniumsalze, Alkylbetaine, oxethylierte Fettalkohole, oxethylierte Nonylphenole, Fettsäurealkanolamide, oxethylierte Fettsäureester, Fettalkoholpolyglykolethersulfate, Alkylpolyglucoside, Verdickungsmittel wie höhere Fettalkohole, Stärke, Cellulosederivate, Vaseline, Paraffinöl, Fettsäuren, und andere Fettkomponenten in emulgierter Form, wasserlöslische polymere Verdikungsmittel wie natürliche Gummen, Guargummi, Xanthangummi, Johannisbrotkernmehl, Pektin, Dextran, Agar-Agar, Amylose, Amylopektin, Dextrine, Tone oder vollsynthetische Hydrokolloide wie zum Beispiel Polyvinylalkohol, sowie außerdem Pflegestoffe wie Lanolinderivate, Cholesterin, Pantothensäure, wasserlöslische kationische Polymere, Proteinderivate, Provitamine, Vitamine, Pflanzenextrakte, Zucker und Betain, Hilfstoffe wie Feuchthaltmittel, Elektrolyte, Antioxidantien, Fettamide, Sequestrierungsmittel, filmbildende Agentien und Konservierungsmittel, enthalten sein. Neben Wasser kann auch ein wasserlösliches organisches Lösungsmittel oder ein Gemisch derartiger Lösungsmittel sowie ein Wasser/Lösungsmittel-Gemisch verwendet werden.
Die erwähnten Bestandteile werden in den für solche Zwecke üblichen Mengen verwendet, zum Beispiel die Netzmittel und Emulgatoren in Konzentrationen von etwa 0,1 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Das erfindungsgemäße Färbemittel weist einen pH-Wert von etwa 3 bis 11, vorzugsweise etwa 3 bis 10 auf. Zur Einstellung des erfindungsgemäßen pH-Wertes sind sowohl organische als auch anorganische Säuren oder Basen geeignet. Als geeignete Säuren sind insbesonders die folgenden Säuren zu nennen: α-Hydroxycarbonsäuren, wie zum Beispiel Glykolsäure, Milchsäure, Weinsäure, Zitronensäure oder Äpfelsäure; Ascorbinsäure; Gluconsäurelacton; Essigsäure; Salzsäure oder Phosphorsäure, sowie Mischungen dieser Säuren. Als geeignete Basen sind insbesonders Natriumcarbonat, Natriumhydrogencarbonat, Kaliumcarbonat, Kaliumhydrogencarbonat, Natriumphosphat, Borax (Na₂B₄O₇ x 10H₂O), Dinatriumhydrogenphosphat, Natriumhydroxid, Kaliumhydroxid, Ammoniak und andere organische Amine wie Monoethanolamin, Diethanonolamin, Triethanolamin, N-Methyl-N-ethanolamin, N-Methyl-N,N-diethanolamin, 2-(2-Hydroxyethoxy)-ethanolamin, Di-2-(2-hydroxyethoxy)-ethanamin und Tri-2-(2-hydroxyethoxy)-ethanamin, zu nennen. Die Einstellung von alkalischen pH-Werten erfolgt vorzugsweise mit Ammoniak und/oder Monoethanolamin.

Die Anwendung des erfindungsgemäßen Färbemittels erfolgt in der Regel indem man eine für die Haarfärbung ausreichende Menge, je nach Haarlänge etwa 30 bis 120 Gramm, des Haarfärbemittels auf das Haar aufträgt, das Haarfärbemittel bei etwa 15 bis 50 Grad Celsius, vorzugsweise 30 bis 40 Grad Celsius, etwa 1 bis 60 Minuten, vorzugsweise 5 bis 30 Minuten, einwirken läßt, das Haar anschließend gründlich mit Wasser ausspült, gegebenenfalls mit einem Shampoo wäscht und abschließend trocknet.

Das nach Anspruch 9 beschriebene Färbemittel enthält für kosmetische Mittel übliche natürliche oder synthetische Polymere beziehungsweise modifizierte Polymere natürlichen Ursprungs, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estern der Polyacrylsäure, Polymethylacrylsäure und Aminoalkoholen beispielsweise deren Salze oder Quaternisierungsprodukte, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (entacetyliertes Chitin) oder Chitosanderivate, eingesetzt werden können.

Die vorgenannten Polymere können in dem erfindungsgemäßen Mittel in der für solche Mittel üblichen Mengen, insbesondere in einer Menge von etwa 1 bis 5 Gewichtsprozent, enthalten. Der pH-Wert des erfindungsgemäßen Tönungsfestigers oder Farbfestigers beträgt vorzugsweise etwa 4 bis 10.

Die Anwendung des Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Das erfindungsgemäße Färbemittel ermöglicht eine hervorragende, gleichmäßige, intensive und dauerhafte rote bis violette Färbung der Keratinfasern, beispielsweise menschlicher Haarn, Wolle oder Pelzen, mit einer besonders guten Lichtechtheit und Schweißbeständigkeit.

Die Farbstoffe der Formel (I) sind zum Teil an sich bekannt. Sie können in Analogie zu bekannten Herstellungsverfahren, wie zum Beispiel via Azokupplung von 2-Aminothiazolderivaten mit Indol-Derivaten, und nachfolgende Quaternisierung hergestellt werden.

Die nachfolgenden Beispiele sollen den Gegenstand der Erfindung näher erläutern, ohne diesen hierauf zu beschränken.

### Beispiele

### Färbebeispiele 1 bis 9

| | |
|---|---|
| 2,5 mmol | Verbindung der Formel (I) gemäß Tabelle 1 |
| 12,5 g | Ethanol |
| 10,0 g | Cetyltrimethylammoniumchlorid |
| ad 100,0 g | Wasser, vollentsalzt |

Die Färbelösung wird gegebenfalls durch Zugabe von Ammoniak oder Zitronensäure auf die erwünschten pH-Werte eingestellt.

Die Haarfärbung erfolgt indem eine für die Haarfärbung ausreichende Menge des Färbemittels auf das Haar aufgetragen wird und mit einem Pinsel gleichmäßig verteilt wird. Nach einer Einwirkungszeit von 30 Minuten bei 40°C wird das Haar mit lauwarmem Wasser ausgespült, mit einem Shampoo gewaschen, mit lauwarmem Wasser ausgespült und sodann getrocknet.

Die Färbeergebnisse sind in der nachfolgenden Tabelle 1 zusammengefaßt.

**Tabelle 1**

| **Bsp.** | **Verbindung der Formel (I)** | **pH Wert des Färbemittels** | **Farbton nach dem Färben** | **Farbmeßwerte nach dem Färben** |
|---|---|---|---|---|
| **1** | 3-Methyl-2-[(1-methyl-2-phenyl-1 H-indol-3-yl)azo]-thiazolium-monomethyl-sulfat | 6,2 | rot | L= 29,61 |
| | | | | a= 47,13 |
| | | | | b= 19,96 |
| **2** | 3,5-Dimethyl-2-[(1-methyl-2-phenyl-1 H-indol-3-yl)azo]-thiazolium-monomethylsulfat | 6,6 | rot-violett | L= 31,15 |
| | | | | a= 46,96 |
| | | | | b= 15,86 |
| **3** | 3,4-Dimethyl-2-[(1-methyl-2-phenyl-1 H-indol-3-yl)azo]-thiazolium-monomethylsulfat | 6,5 | rot-violett | L= 27,93 |
| | | | | a= 42,70 |
| | | | | b= 14,21 |
| **4** | 3,4,5-Trimethyl-2-[(1-methyl-2-phenyl-1 H-indol-3-yl)azo]-thiazolium-monomethylsulfat | 6,6 | violett | L=25,42 |
| | | | | a= 37,65 |
| | | | | b= 6,95 |
| **5** | 5-Brom-3-methyl-2-[(1-methyl-2-phenyl-1 H-indol-3-yl)azo]-thiazolium-monomethylsulfat | 5,9 | violett-rosa | L= 35,87 |
| | | | | a= 46,89 |
| | | | | b= 4,09 |
| **6** | 5-Methoxy-3-methyl-2-[(1-methyl-2-phenyl-1 H-indol-3-yl)azo]-thiazolium-monomethylsulfat | 7,3 | violett-aubergine | L= 24,17 |
| | | | | a= 36,57 |
| | | | | b= 1,55 |
| **7** | 3-Methyl-2-[(2-phenyl-1H-indol-3-yl)azo]-thiazolium-monomethylsulfat | 3,2 | rot | L= 42,66 |
| | | | | a= 36,46 |
| | | | | b= 16,15 |
| **8** | 3-Methoxy-2-[(2-phenyl-1H-indol-3-yl)azo]-thiazoliummonomethylsulfat | 3,1 | rosa | L= 61,06 |
| | | | | a= 25,74 |
| | | | | b= 8,08 |
| **9** | 3-Methyl-2-[(1,2-diphenyl-1 H-indol-3-yl)azo]-thiazolium-monomethylsulfat | 6,2 | rot | L= 28,51 |
| | | | | a= 45,89 |
| | | | | b= 17,46 |

### Färbebeispiele 10 bis 15

| | |
|---|---|
| 0,625 mmol | Verbindung der Formel (I) gemäß Tabelle 2 |
| 0,625 mmol | kationischer Farbstoff gemäß Tabelle 2 |
| 5,0 g | Ethanol |
| 4,0 g | Decylglucosid |
| 0,2 g | Ethylendiaminotetraessigsäure-Dinatriumsalz |
| ad 100,0 g | Wasser, vollentsalzt |

Die Färbelösung wird gegebenfalls durch Zugabe von Ammoniak oder Zitronensäure auf die gewünschten pH-Werte eingestellt.

Die Haarfärbung erfolgt indem eine für die Haarfärbung ausreichende Menge des Färbemittels auf das Haar aufgetragen wird und mit einem Pinsel gleichmäßig verteilt wird. Nach einer Einwirkungszeit von 30 Minuten bei 40 °C wird das Haar mit lauwarmem Wasser ausgespült, mit einem Shampoo gewaschen, mit laufwarmem Wasser ausgespült und sodann getrocknet.
Die Einsatzmengen der Farbstoffe sowie die Färbeergebnisse sind in der nachfolgenden Tabelle 2 zusammengefaßt.

**Tabelle 2**

| **Bsp.** | **Verbindung der Formel (I) /kationischer Farbstoff** | **pH Wert des Färbemittels** | **Farbton nach dem Färben** | **Farbmeßwerte nach dem Färben** |
|---|---|---|---|---|
| **10** | 3-Methyl-2-[(1-methyl-2-phenyl-1 H-indol-3-yl)azo]-thiazolium- monomethylsulfat (0,27 g) Basic Brown 17 (0,25 g) | 7,3 | rot-orange | L= 29,07 |
| | | | | a= 42,07 |
| | | | | b= 19,43 |
| **11** | 3-Methyl-2-[(1-methyl-2-phenyl-1 H-indol-3-yl)azo]-thiazolium- monomethylsulfat (0,27 g) Basic Brown No.16 (0,22 g) | 7,2 | braun-rot | L= 24,32 |
| | | | | a= 25,51 |
| | | | | b= 12,99 |
| **12** | 3-Methyl-2-[(1-methyl-2-phenyl-1 H-indol-3-yl)azo]-thiazolium- monomethylsulfat (0,27 g) Basic Yellow No.57 (0,23 g) | 7,1 | rot-orange | L= 31,10 |
| | | | | a= 46,60 |
| | | | | b= 23,20 |
| **13** | 3-Methyl-2-[(1-methyl-2-phenyl-1 H-indol-3-yl)azo]-thiazolium- monomethylsulfat (0,27 g) Basic Blue No.99 (0,28 g) | 7,1 | braun-violett | L= 20,73 |
| | | | | a= 11,10 |
| | | | | b= 4,80 |
| **14** | 3-Methyl-2-[(1-methyl-2-phenyl-1 H-indol-3-yl)azo]-thiazolium- monomethylsulfat (0,27 g) Basic Violet No.2 (0,23 g) | 7,3 | violett-rot | L= 26,46 |
| | | | | a= 43,82 |
| | | | | b= 15,49 |
| **15** | 3-Methyl-2-[(1-methyl-2-phenyl-1 H-indol-3-yl)azo]-thiazolium- monomethylsulfat (0,27 g) Basic Brown 17 (0,25 g) Basic Yellow No.57 (0,23 g) Basic Blue No.99 (0,28 g) | 7,1 | braun | L= 25,22 |
| | | | | a= 17,27 |
| | | | | b= 10,17 |

### Färbebeispiel 16

| | |
|---|---|
| 2,5 mmol | Farbstoff der Formel (I) |
| 5,0 g | Ethanol |
| 4,0 g | Decylglucosid |
| 0,2 g | Ethylendiaminotetraessigsäure-Dinatriumsalz |
| ad 100,0 g | Wasser, vollentsalzt |

Die Färbelösung wird gegebenfalls durch Zugabe von Ammoniak oder Zitronensäure auf die erwünschten pH-Wert eingestellt.

Die Haarfärbung erfolgt indem eine für die Haarfärbung ausreichende Menge des Färbemittels auf das Haar aufgetragen wird und mit einem Pinsel gleichmäßig verteilt wird. Nach einer Einwirkungszeit von 30 Minuten bei 40 °C wird das Haar mit lauwarmem Wasser ausgespült, mit einem Shampoo gewaschen, mit lauwarmem Wasser ausgespült und sodann getrocknet.

Die Färbeergebnisse sind in der nachfolgenden Tabelle 3 zusammengefasst.

**Tabelle 3**

| **Bsp.** | **Verbindung der Formel (I)** | **pH Wert des Färbemittels** | **Farbton nach dem Färben** | **Farbmeßwerte nach dem Färben** |
|---|---|---|---|---|
| **16** | 3-Methyl-2-[(1-methyl-2-phenyl-1 H-indol-3-yl)azo]-thiazolium-monomethylsulfat | 7,5 | rot | L= 31,7 |
| | | | | a= 48,8 |
| | | | | b= 23,8 |

### Färbebeispiel 17

| | |
|---|---|
| 2,5 mmol | Farbstoff der Formel (I) |
| 5,0 g | Ethanol |
| 7,5 g | Tegobetain |
| ad 100,0 g | Wasser, vollentsalzt |

Die Färbelösung wird gegebenfalls durch Zugabe von Ammoniak auf die erwünschten pH-Wert eingestellt.

Die Haarfärbung erfolgt indem eine für die Haarfärbung ausreichende Menge des Färbemittels auf das Haar aufgetragen wird und mit einem Pinsel gleichmäßig verteilt wird. Nach einer Einwirkungszeit von 30 Minuten bei 40 °C wird das Haar mit lauwarmem Wasser ausgespült, mit einem Shampoo gewaschen, mit lauwarmem Wasser ausgespült und sodann getrocknet.

Die Färbeergebnisse sind in der nachfolgenden Tabelle 4 zusammengefasst.

**Tabelle 4**

| **Bsp.** | **Verbindung der Formel (I)** | **pH Wert des Färbemittels** | **Farbton nach dem Färben** | **Farbmeßwerte nach dem Färben** |
|---|---|---|---|---|
| 17 | 3-Methyl-2-[(1-methyl-2-phenyl-1 H-indol-3-yl)azo]-thiazolium-monomethylsulfat | 9,7 | rot | L= 29,97 |
| | | | | a= 42,76 |
| | | | | b= 19,45 |

### Färbebeispiel 18

| | |
|---|---|
| 2,5 mmol | Farbstoff der Formel (I) |
| 5,0 g | Ethanol |
| 7,5 g | Laurylethersulfat 28% in Wasser |
| ad 100,0 g | Wasser, vollentsalzt |

Die Färbelösung wird gegebenfalls durch Zugabe von Ammoniak auf die erwünschten pH-Wert eingestellt.

Die Haarfärbung erfolgt indem eine für die Haarfärbung ausreichende Menge des Färbemittels auf das Haar aufgetragen wird und mit einem Pinsel gleichmäßig verteilt wird. Nach einer Einwirkungszeit von 30 Minuten bei 40 °C wird das Haar mit lauwarmem Wasser ausgespült, mit einem Shampoo gewaschen, mit lauwarmem Wasser ausgespült und sodann getrocknet.

Die Färbeergebnisse sind in der nachfolgenden Tabelle 5 zusammengefasst.

**Tabelle 5**

| **Bsp.** | **Verbindung der Formel (I)** | **pH Wert des Färbemittels** | **Farbton nach dem Färben** | **Farbmeßwerte nach dem Färben** |
|---|---|---|---|---|
| **18** | 3-Methyl-2-[(1-methyl-2-phenyl-1 H-indol-3-yl)azo]-thiazolium-monomethylsulfat | 9,0 | blass rot | L= 50,44 |
| | | | | a= 42,03 |
| | | | | b= 18,67 |

Die in den vorliegenden Beispielen angegebenen L*a*b*-Farbmesswerte wurden mit einem Farbmessgerät der Firma Minolta, Typ Chromameter II, ermittelt. Hierbei steht der L-Wert für die Helligkeit (das heißt je geringer der L-Wert ist, umso größer ist die Farbintensität), während der α-Wert ein Maß für den Rotanteil ist (das heißt je größer der α-Wert ist, umso größer ist der Rotanteil). Der b-Wert ist ein Maß für den Blauanteil der Farbe, wobei der Blauanteil umso größer ist, je negativer der b-Wert ist.

Alle Prozentangaben in der vorliegenden Anmeldung stellen, sofern nicht anders angegeben, Gewichtsprozente dar.

## Patentansprüche

1. Verwendung eines Mittels, welches mindestens einen Indolylthiazoliumazofarbstoff der allgemeinen Formel (I) enthält, 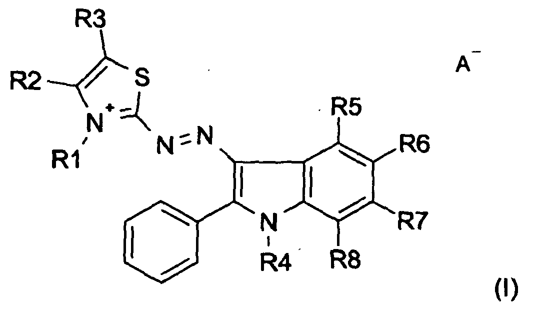 wobei
**R1** eine gesättigte oder ungesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkyl-gruppe, eine Di-(C₁-G₆)-alkylamino-(C₁-C₁₂)-alkylgruppe, eine Cyano-(C₁-C₁₂)-alkylgruppe, eine substituierte oder unsubstituierte Phenylgruppe oder eine substituierte oder unsubstituierte Benzylgruppe darstellt,
**R2** und **R3** gleich oder verschieden sein können und unabhängig voneinander Wasserstoff, ein Halogenatom, eine gesättigte oder ungesättigte (C₁-C₁₂)-Alkylgruppe, eine (C₁-C₁₂)-Alkoxygruppe, eine Nitrogruppe, eine Aminogruppe, eine (C₁-C₁₂)-Alkylaminogruppe, eine Di(C₁-C₁₂)-alkylaminogruppe, eine (C₁-C₁₂)-Hydroxyalkylaminogruppe, eine Di(C₁-C₁₂)-hydroxyalkylaminogruppe, eine substituierte oder unsubstituierte Phenylgruppe oder eine substituierte oder unsubstituierte Heteroarylgruppe darstellen;
**R4** Wasserstoff, eine gesättigte oder ungesättigte (C₁-C₁₂)-Alkylgruppe, eine mit einem Halogenatom substituierte (C₁-C₁₂)-Alkyl-gruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)Alkylamino-(C₁-C₁₂)-alkylgruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe, eine substituierte oder unsubstituierte Benzylgruppe oder eine substituierte oder unsubstituierte Heteroarylgruppe darstellt;
**R5, R6, R7,** und **R8** gleich oder verschieden sein können und unabhängig voneinander Wasserstoff, ein Halogenatom, eine gesättigte oder ungesättigte (C₁-C₁₂)-Alkylgruppe, eine Hydroxygruppe, eine (C₁-C₁₂)-Alkoxygruppe, eine Nitrogruppe, eine Aminogruppe, eine (C₁-C₁₂)-Alkylaminogruppe oder eine Di(C₁-C₁₂)-alkylaminogruppe darstellen; und
**A⁻** gleich einem Anion einer organischen oder anorganischen Säure ist; zum Färben von Keratinfasern.

2. Verwendung nach Anspruch 1, **dadurch gekennzeichnet, dass R1** gleich einer gesättigten oder ungesättigten (C₁-C₁₂)-Alkylgruppe ist und **R4** gleich Wasserstoff, einer gesättigten oder ungesättigten (C₁-C₁₂)-Alkylgruppe, einer substituierten oder unsubstituierten Phenylgruppe ist.

3. Verwendung nach Anspruch 2, **dadurch gekennzeichnet, dass R1** gleich einer gesättigten (C₁-C₁₂)-Alkylgruppe und **R4** gleich Wasserstoff, einer gesättigten (C₁-C₁₂)-Alkylgruppe, oder einer unsubstituierten Phenylgruppe ist.

4. Verwendung nach einem der Ansprüche 1 bis 3, **dadurch gekennzeichnet, dass A⁻** gleich einem Chlorid-, Bromid-, Jodid-, Hydrogensulfat-, Sulfat-, Toluolsulfonat-, Benzolsulfonat-, Monomethylsulfat-, Hexafluorphosphat-, Hexafluorantimonat-, Tetrafluorborat-, Tetraphenylborat-, Formiat-, Acetat- oder Propionat-Anion ist.

5. Verwendung nach einem der Ansprüche 1 bis 4, **dadurch gekennzeichnet, dass** die Verbindung der Formel (I) ausgewählt ist aus 3-Methyl-2-[(1-methyl-2-phenyl-1H-indol-3-yl)azo]-thiazolium-chlorid, 3-Methyl-2-[(1-methyl-2-phenyl-1H-indol-3-yl)azo]-thiazolium-bromid, 3-Methyl-2-[(1-methyl-2-phenyl-1H-indol-3-yl)azo]-thiazolium-monomethylsulfat, 3,4-Dimethyl-2-[(1-methyl-2-phenyl-1H-indol-3-yl)azo]-thiazolium-chlorid, 3,4-Dimethyl-2-[(1-methyl-2-phenyl-1H-indol-3-yl)azo]-thiazolium-bromid, 3,4-Dimethyl-2-[(1-methyl-2-phenyl-1H-indol-3-yl)azo]-thiazolium-mono-methylsulfat, 3,5-Dimethyl-2-[(1-methyl-2-phenyl-1H-indol-3-yl)azo]-thiazolium-chlorid, 3,5-Dimethyl-2-[(1-methyl-2-phenyl-1 H-indol-3-yl)azo]-thiazolium-bromid, 3,5-Dimethyl-2-[(1-methyl-2-phenyl-1H-indol-3-yl)azo]-thiazolium-monomethylsulfat, 3,4,5-Trimethyl-2-[(1-methyl-2-phenyl-1 H-indol-3-yl)azo]-thiazolium-chlorid, 3,4,5-Trimethyl-2-[(1-methyl-2-phenyl-1 H-indol-3-yl)azo]-thiazolium-bromid, 3,4,5-Trimethyl-2-[(1-methyl-2-phenyl-1 H-indol-3-yl)azo]-thiazolium-monomethylsulfat, 5-Brom-3-methyl-2-[(1-methyl-2-phenyl-1 H-indol-3-yl)azo]-thiazoliumchlorid, 5-Brom-3-methyl-2-[(1-methyl-2-phenyl-1 H-indol-3-yl)azo]-thiazolium-bromid, 5-Brom-3-methyl-2-[(1-methyl-2-phenyl-1 H-indol-3-yl)azo]-thiazolium- monomethylsulfat, 5-Methoxy-3-methyl-2-[(1-methyl-2-phenyl-1 H-indol-3-yl)azo]-thiazolium-chlorid, 5-Methoxy-3-methyl-2-[(1-methyl-2-phenyl-1 H-indol-3-yl)azo]-thiazolium-bromid, 5-Methoxy-3-methyl-2-[(1-methyl-2-phenyl-1H-indol-3-yl)azo]-thiazolium- monomethylsulfat, 3-Methyt-2-[(2-phenyl-1H-indol-3-yl)azo]-thiazolium-chlorid, 3-Methyl-2-[(2-phenyl-1 H-indol-3-yl)azo]-thiazolium-bromid, 3-Methyl-2-[(2-phenyl-1H-indol-3-yl)azo]-thiazolium-monomethylsulfat, 3,4-Dimethyl-2-[(2-phenyl-1H-indol-3-yl)azo]-thiazolium-chlorid, 3,4-Dimethyl-2-[(2-phenyl-1 H-indol-3-yl)azo]-thiazolium-bromid, 3,4-Dimethyl-2-[(2-phenyl-1 H-indol-3-yl)azo]-thiazolium-monomethylsulfat, 3,5-Dimethyl-2-[(2-phenyl-1 H-indol-3-yl)azo]-thiazolium-chlorid, 3,5-Dimethyl-2-[(2-phenyl-1H-indol-3-yl)azo]-thiazolium-bromid, 3,5-Dimethyl-2-[(2-phenyl-1 H-indol-3-yl)azo]-thiazolium-monomethylsulfat, 3,4,5-Trimethyl-2-[(2-phenyl-1 H-indol-3-yl)azo]-thiazolium-chlorid, 3,4,5-Trimethyl-2-[(2-phenyl-1 H-indol-3-yl)azo]-thiazolium-bromid, 3,4,5-Trimethyl-2-[(2-phenyl-1 H-indol-3-yl)azo]-thiazolium-monomethylsulfat, 5-Brom-3-methyl-2-[(2-phenyl-1H-indol-3-yl)azo]-thiazolium-chlorid, 5-Brom-3-methyl-2-[(2-phenyl-1 H-indol-3-yl)azo]-thiazolium-bromid, 5-Brom-3-methyl-2-[(2-phenyl-1 H-indol-3-yl)azo]-thiazolium-monomethylsulfat, 5-Methoxy-3-methyl-2-[(2-phenyl-1H-indol-3-yl)azo]-thiazolium-chlorid, 5-Methoxy-3-methyl-2-[(2-phenyl-1H-indol-3-yl)azo]-thiazolium-bromid, 5-Methoxy-3-methyl-2-[(2-phenyl-1H-indol-3-yl)azo]-thiazolium-monomethylsulfat, 3-Methyl-2-[(1,2-diphenyl-1 H-indol-3-yl)azo]-thiazolium-chlorid, 3-Methyl-2-[(1,2-diphenyl-1 H-indol-3-yl)azo]-thiazolium-bromid, 3-Methyl-2-[(1,2-diphenyl-1 H-indol-3-yl)azo]-thiazolium-monomethylsulfat, 3,4-Dimethyl-2-[(1,2-diphenyl-1 H-indol-3-yl)azo]-thiazolium-chlorid, 3,4-Dimethyl-2-[(1,2-diphenyl-1 H-indol-3-yl)azo]-thiazolium-bromid, 3,4-Dimethyl-2-[(1,2-diphenyl-1H-indol-3-yl)azo]-thiazolium-monomethylsulfat, 3,5-Dimethyl-2-[(1,2-diphenyl-1 H-indol-3-yl)azo]-thiazolium-chlorid, 3,5-Dimethyl-2-[(1,2-diphenyl-1 H-indol-3-yl)azo]-thiazolium-bromid, 3,5-Dimethyl-2-[(1,2-diphenyl-1 H-indol-3-yl)azo]-thiazolium-monomethylsulfat, 3,4,5-Trimethyl-2-[(1,2-diphenyl-1H-indol-3-yl)azo]-thiazolium-chlorid, 3,4,5-Trimethyl-2-[(1,2-diphenyl-1 H-indol-3-yl)azo]-thiazolium-bromid, 3,4,5-Trimethyl-2-[(1,2-diphenyl-1H-indol-3-yl)azo]-thiazolium-monomethylsulfat, 5-Brom-3-methyl-2-[(1,2-diphenyl-1 H-indol-3-yl)azo]-thiazolium-chlorid, 5-Brom-3-methyl-2-[(1,2-diphenyl-1H-indol-3-yl)azo]-thiazolium-bromid, 5-Brom-3-methyl-2-[(1,2-diphenyl-1 H-indol-3-yl)azo]-thiazolium-monomethylsulfat, 5-Methoxy-3-methyl-2-[(1,2-diphenyl-1 H-indol-3-yl)azo]-thiazolium-chlorid, 5-Methoxy-3-methyl-2-[(1,2-diphenyl-1 H-indol-3-yl)azo]-thiazolium-bromid und 5-Methoxy-3-methyl-2-[(1,2-diphenyl-1H-indol-3-yl)azo]-thiazolium-mono-methylsulfat.

6. Mittel zum Färben von Keratinfasern, **dadurch gekennzeichnet, dass** es mindestens einen Indolylthiazoliumazofarbstoff der allgemeinen Formel (I) sowie mindestens einen weiteren direktziehende Farbstoff enthält, 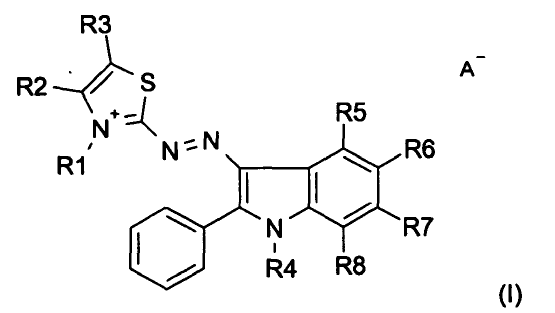 wobei
**R1** eine gesättigte oder ungesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkyl-gruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe oder eine substituierte oder unsubstituierte Benzylgruppe darstellt,
R2 und R3 gleich oder verschieden sein können und unabhängig voneinander Wasserstoff, ein Halogenatom, eine gesättigte oder ungesättigte (C₁-C₁₂)-Alkylgruppe, eine (C₁-C₁₂)-Alkoxygruppe, eine Nitrogruppe, eine Aminogruppe, eine (C₁-C₁₂)-Alkylaminogruppe, eine Di(C₁-C₁₂)-alkylaminogruppe, eine (C₁-C₁₂)-Hydroxyalkylaminogruppe, eine Di(C₁-C₁₂)-hydroxyalkylaminogruppe, eine substituierte oder unsubstituierte Phenylgruppe oder eine substituierte oder unsubstituierte Heteroarylgruppe darstellen;
**R4** Wasserstoff, eine gesättigte oder ungesättigte (C₁-C₁₂)-Alkylgruppe, eine mit einem Halogenatom substituierte (C₁-C₁₂)-Alkyl-gruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkylgruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe, eine substituierte oder unsubstituierte Benzylgruppe oder eine substituierte oder unsubstituierte Heteroarylgruppe darstellt;
**R5, R6, R7,** und **R8** gleich oder verschieden sein können und unabhängig voneinander Wasserstoff, ein Halogenatom, eine gesättigte oder ungesättigte (C₁-C₁₂)-Alkylgruppe, eine Hydroxygruppe, eine (C₁-C₁₂)-Alkoxygruppe, eine Nitrogruppe, eine Aminogruppe, eine (C₁-C₁₂)-Alkylaminogruppe oder eine Di(C₁-C₁₂)-alkylaminogruppe darstellen; und
**A'** gleich einem Anion einer organischen oder anorganischen Säure ist.

7. Mittel nach Anspruch 6, **dadurch gekennzeichnet, dass** der weitere direktziehende Farbstoff in einer Gesamtmenge von 0,01 bis 4 Gewichtsprozent enthalten ist.

8. Mittel zum Färben von Keratinfasern, **dadurch gekennzeichnet, dass** es mindestens einen Indolylthiazoliumazofarbstoff der allgemeinen Formel (I) sowie mindestens eine zusätzliche Oxidationsfarbstoffvorstufe enthält und vor der Anwendung mit einem Oxidationsmittel vermischt wird, 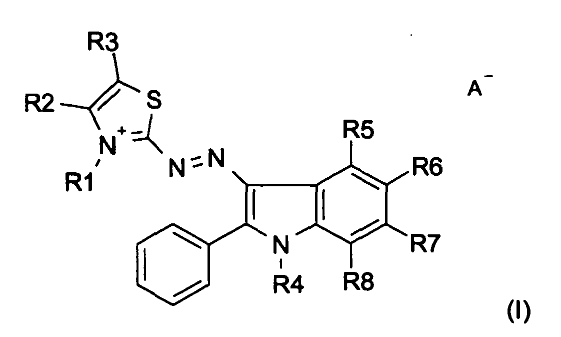 wobei
**R1** eine gesättigte oder ungesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkyl-gruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe oder eine substituierte oder unsubstituierte Benzylgruppe darstellt,
**R2** und **R3** gleich oder verschieden sein können und unabhängig voneinander Wasserstoff, ein Halogenatom, eine gesättigte oder ungesättigte (C₁-C₁₂)-Alkylgruppe, eine (C₁-C₁₂)-Alkoxygruppe, eine Nitrogruppe, eine Aminogruppe, eine (C₁-C₁₂)-Alkylaminogruppe, eine Di(C₁-C₁₂)-alkylaminogruppe, eine (C₁-C₁₂)-Hydroxyalkylaminogruppe, eine Di(C₁-C₁₂)-hydroxyalkylaminogruppe, eine substituierte oder unsubstituierte Phenylgruppe oder eine substituierte oder unsubstituierte Heteroarylgruppe darstellen;
**R4** Wasserstoff, eine gesättigte oder ungesättigte (C₁-C₁₂)-Alkylgruppe, eine mit einem Halogenatom substituierte (C₁-C₁₂)-Alkyl-gruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkylgruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe, eine substituierte oder unsubstituierte Benzylgruppe oder eine substituierte oder unsubstituierte Heteroarylgruppe darstellt;
**R5, R6, R7,** und **R8** gleich oder verschieden sein können und unabhängig voneinander Wasserstoff, ein Halogenatom, eine gesättigte oder ungesättigte (C₁-C₁₂)-Alkylgruppe, eine Hydroxygruppe, eine (C₁-C₁₂)-Alkoxygruppe, eine Nitrogruppe, eine Aminogruppe, eine (C₁-C₁₂)-Alkylaminogruppe oder eine Di(C₁-C₁₂)-alkylaminogruppe darstellen; und
**A⁻** gleich einem Anion einer organischen oder anorganischen Säure ist.

9. Mittel zum Färben von Keratinfasern, **dadurch gekennzeichnet, dass** es mindestens einen Indolylthiazoliumazofarbstoff der allgemeinen Formel (I) sowie mindestens ein für kosmetische Mittel übliches natürliches Polymer, synthetisches Polymer oder modifiziertes Polymer natürlichen Ursprungs enthält und in Form eines Tönungsfestigers oder Farbfestigers vorliegt, 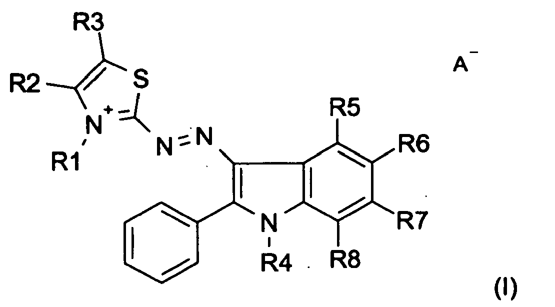 wobei
**R1** eine gesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkyl-gruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe oder eine substituierte oder unsubstituierte Benzylgruppe darstellt,
**R2** und **R3** gleich oder verschieden sein können und unabhängig voneinander Wasserstoff, ein Halogenatom, eine gesättigte oder ungesättigte (C₁-C₁₂)-Alkylgruppe, eine (C₁-C₁₂)-Alkoxygruppe, eine Nitrogruppe, eine Aminogruppe, eine (C₁-C₁₂)-Alkylaminogruppe, eine Di(C₁-C₁₂)-alkylaminogruppe, eine (C₁-C₁₂)-Hydroxyalkylaminogruppe, eine Di(C₁-C₁₂)-hydroxyalkylaminogruppe, eine substituierte oder unsubstituierte Phenylgruppe oder eine substituierte oder unsubstituierte Heteroarylgruppe darstellen;
**R4** Wasserstoff, eine gesättigte oder ungesättigte (C₁-C₁₂)-Alkylgruppe, eine mit einem Halogenatom substituierte (C₁-C₁₂)Alkyl-gruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkylgruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe, eine substituierte oder unsubstituierte Benzylgruppe oder eine substituierte oder unsubstituierte Heteroarylgruppe darstellt;
**R5, R6, R7,** und **R8** gleich oder verschieden sein können und unabhängig voneinander Wasserstoff, ein Halogenatom, eine gesättigte oder ungesättigte (C₁-C₁₂)-Alkylgruppe, eine Hydroxygruppe, eine (C₁-C₁₂)-Alkoxygruppe, eine Nitrogruppe, eine Aminogruppe, eine (C₁-C₁₂)-Alkylaminogruppe oder eine Di(C₁-C₁₂)-alkylaminogruppe darstellen; und
**A'** gleich einem Anion einer organischen oder anorganischen Säure ist.

10. Mittel nach einem der Ansprüche 6 bis 8, **dadurch gekennzeichnet, dass R1** gleich einer gesättigten oder ungesättigten (C₁-C₁₂)-Alkylgruppe ist und **R4** gleich Wasserstoff, einer gesättigten oder ungesättigten (C₁-C₁₂)-Alkylgruppe, einer substituierten oder unsubstituierten Phenylgruppe ist.

11. Mittel nach einem der Ansprüche 6 bis 10, **dadurch gekennzeichnet, dass R1** gleich einer gesättigten (C₁-C₁₂)-Alkylgruppe und **R4** gleich Wasserstoff, einer gesättigten (C₁-C₁₂)-Alkylgruppe, oder einer unsubstituierten Phenylgruppe ist.

12. Mittel nach einem der Ansprüche 6 bis 11, **dadurch gekennzeichnet, dass A⁻** gleich einem Chlorid-, Bromid-, Jodid-, Hydrogensulfat-, Sulfat-, Toluolsulfonat-, Benzolsulfonat-, Monomethylsulfat-, Hexafluorphosphat-, Hexafluorantimonat-, Tetrafluorborat-, Tetraphenylborat-, Formiat-, Acetat- oder Propionat-Anion ist.

13. Mittel nach einem der Ansprüche 6 bis 12, **dadurch gekennzeichnet, dass** die Verbindung der Formel (I) ausgewählt ist aus 3-Methyl-2-[(1-methyl-2-phenyl-1 H-indol-3-yl)azo]-thiazolium-chlorid, 3-Methyl-2-[(1-methyl-2-phenyl-1 H-indol-3-yl)azo]-thiazolium-bromid, 3-Methyl-2-[(1-methyl-2-phenyl-1H-indol-3-yl)azo]-thiazolium-monomethylsulfat, 3,4-Dimethyl-2-[(1-methyl-2-phenyl-1H-indol-3-yl)azo]-thiazolium-chlorid, 3,4-Dimethyl-2-[(1-methyl-2-phenyl-1 H-indol-3-yl)azo]-thiazolium-bromid, 3,4-Dimethyl-2-[(1-methyl-2-phenyl-1 H-indol-3-yl)azo]-thiazolium-mono-methylsulfat, 3,5-Dimethyl-2-[(1-methyl-2-phenyl-1 H-indol-3-yl)azo]-thiazolium-chlorid, 3,5-Dimethyl-2-[(1 -methyl-2-phenyl-1 H-indol-3-yl)azo]-thiazolium-bromid, 3,5-Dimethyl-2-[(1-methyl-2-phenyl-1 H-indol-3-yl)azo]-thiazolium-monomethylsulfat, 3,4,5-Trimethyl-2-[(1-methyl-2-phenyl-1 H-indol-3-yl)azo]-thiazolium-chlorid, 3,4,5-Trimethyl-2-[(1-methyl-2-phenyl-1 H-indol-3-yl)azo]-thiazolium-bromid, 3,4,5-Trimethyl-2-[(1-methyl-2-phenyl-1 H-indol-3-yl)azo]-thiazolium-monomethylsulfat, 5-Brom-3-methyl-2-[(1-methyl-2-phenyl-1H-indol-3-yl)azo]-thiazolium-chlorid, 5-Brom-3-methyl-2-[(1-methyl-2-phenyl-1H-indol-3-yl)azo]-thiazolium-bromid, 5-Brom-3-methyl-2-[(1-methyl-2-phenyl-1H-indol-3-yl)azo]-thiazolium- monomethylsulfat, 5-Methoxy-3-methyl-2-[(1-methyl-2-phenyl-1 H-indol-3-yl)azo]-thiazolium-chlorid, 5-Methoxy-3-methyl-2-[(1-methyl-2-phenyl-1H-indol-3-yl)azo]-thiazolium-bromid, 5-Methoxy-3-methyl-2-[(1-methyl-2-phenyl-1H-indol-3-yl)azo]-thiazolium- monomethylsulfat, 3-Methyl-2-[(2-phenyl-1 H-indol-3-yl)azo]-thiazolium-chlorid, 3-Methyl-2-[(2-phenyl-1 H-indol-3-yl)azo]-thiazolium-bromid, 3-Methyl-2-[(2-phenyl-1 H-indol-3-yl)azo]-thiazolium-monomethylsulfat, 3,4-Dimethyl-2-[(2-phenyl-1 H-indol-3-yl)azo]-thiazolium-chlorid, 3,4-Dimethyl-2-[(2-phenyl-1 H-indol-3-yl)azo]-thiazolium-bromid, 3,4-Dimethyl-2-[(2-phenyl-1 H-indol-3-yl)azo]-thiazolium-monomethylsulfat, 3,5-Dimethyl-2-[(2-phenyl-1 H-indol-3-yl)azo]-thiazolium-chlorid, 3,5-Dimethyl-2-[(2-phenyl-1 H-indol-3-yl)azo]-thiazolium-bromid, 3,5-Dimethyl-2-[(2-phenyl-1 H-indol-3-yl)azo]-thiazolium-monomethylsulfat, 3,4,5-Trimethyl-2-[(2-phenyl-1 H-indol-3-yl)azo]-thiazolium-chlorid, 3,4,5-Trimethyl-2-[(2-phenyl-1H-indol-3-yl)azo]-thiazolium-bromid, 3,4,5-Trimethyl-2-[(2-phenyl-1 H-indol-3-yl)azo]-thiazolium-monomethylsulfat, 5-Brom-3-methyl-2-[(2-phenyl-1 H-indol-3-yl)azo]-thiazolium-chlorid, 5-Brom-3-methyl-2-[(2-phenyl-1 H-indol-3-yl)azo]-thiazolium-bromid, 5-Brom-3-methyl-2-[(2-phenyl- H-indol-3-yl)azo]-thiazolium-monomethylsulfat, 5-Methoxy-3-methyl-2-[(2-phenyl-1H-indol-3-yl)azo]-thiazolium-chlorid, 5-Methoxy-3-methyl-2-[(2-phenyl-1 H-indol-3-yl)azo]-thiazolium-bromid, 5-Methoxy-3-methyl-2-[(2-phenyl-1 H-indol-3-yl)azo]-thiazolium-monomethylsulfat, 3-Methyl-2-[(1,2-diphenyl-1H-indol-3-yl)azo]-thiazolium-chlorid, 3-Methyl-2-[(1,2-diphenyl-1H-indol-3-yl)azo]-thiazolium-bromid, 3-Methyl-2-[(1,2-diphenyl-1H-indol-3-yl)azo]-thiazolium-monomethylsulfat, 3,4-Dimethyl-2-[(1,2-diphenyl-1H-indol-3-yl)azo]-thiazolium-chlorid, 3,4-Dimethyl-2-[(1,2-diphenyl-1 H-indol-3-yl)azo]-thiazolium-bromid, 3,4-Dimethyl-2-[(1,2-diphenyl-1H-indol-3-yl)azo]-thiazolium-monomethylsulfat, 3,5-Dimethyl-2-[(1,2-diphenyl-1 H-indol-3-yl)azo]-thiazolium-chlorid, 3,5-Dimethyl-2-[(1,2-diphenyl-1H-indol-3-yl)azo]-thiazolium-bromid, 3,5-Dimethyl-2-[(1,2-diphenyl-1 H-indol-3-yl)azo]-thiazolium-monomethylsulfat, 3,4,5-Trimethyl-2-[(1,2-diphenyl-1 H-indol-3-yl)azo]-thiazolium-chlorid, 3,4,5-Trimethyl-2-[(1,2-diphenyl-1 H-indol-3-yl)azo]-thiazolium-bromid, 3,4,5-Trimethyl-2-[(1,2-diphenyl-1H-indol-3-yl)azo]-thiazolium-monomethylsulfat, 5-Brom-3-methyl-2-[(1,2-diphenyl-1 H-indol-3-yl)azo]-thiazolium-chlorid, 5-Brom-3-methyl-2-[(1,2-diphenyl-1H-indol-3-yl)azo]-thiazolium-bromid, 5-Brom-3-methyl-2-[(1,2-diphenyl-1 H-indol-3-yl)azo]-thiazolium-monomethylsulfat, 5-Methoxy-3-methyl-2-[(1,2-diphenyl-1 H-indol-3-yl)azo]-thiazolium-chlorid, 5-Methoxy-3-methyl-2-[(1,2-diphenyl-1 H-indol-3-yl)azo]-thiazolium-bromid und 5-Methoxy-3-methyl-2-[(1,2-diphenyl-1H-indol-3-yl)azo]-thiazolium-mono-methylsulfat.

14. Mittel nach einem der Ansprüche 6 bis 13, **dadurch gekennzeichnet, dass** die Verbindung der Formel (I) in einer Menge von 0,01 bis 10 Gewichtsprozent enthalten ist.

15. Mittel nach einem der Ansprüche 6 bis 14, **dadurch gekennzeichnet, dass** es ein Haarfärbemittel ist.

## Claims

1. Use of an agent which comprises at least one indolylthiazolium azo dye of the general formula (I), 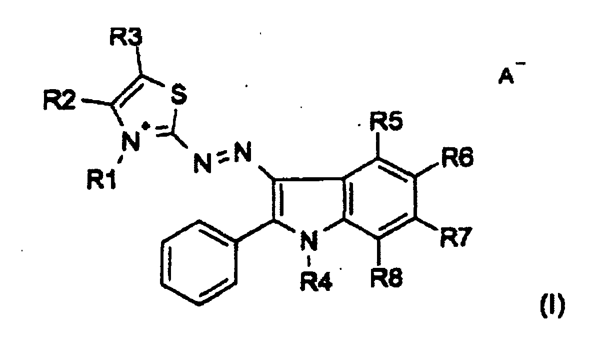 where
**R1** is a saturated or unsaturated (C₁-C₁₂)-alkyl group, a halogen-substituted (C₁-C₁₂)-alkyl group, a hydroxyl-(C₁-C₁₂)-alkyl group, a (C₁-C₆)-alkoxy-(C₁-C₁₂)-alkyl group, an amino-(C₁-C₁₂)-alkyl group, a (C₁-C₆)-alkylamino-(C₁-C₁₂)-alkyl group, a di(C₁-C₆)-alkylamino-(C₁-C₁₂)-alkyl group, a cyano-(C₁-C₁₂)-alkyl group, a substituted or unsubstituted phenyl group or a substituted or unsubstituted benzyl group,
R2 and **R3** may be identical or different and, independently of one another, are hydrogen, a halogen atom, a saturated or unsaturated (C₁-C₁₂)-alkyl group, a (C₁-C₁₂)-alkoxy group, a nitro group, an amino group, a (C₁-C₁₂)-alkylamino group, a di(C₁-C₁₂)-alkylamino group, a (C₁-C₁₂) -hydroxyalkylamino group, a di(C₁-C₁₂)-hydroxyalkylamino group, a substituted or unsubstituted phenyl group or a substituted or unsubstituted heteroaryl group;
R4 is hydrogen, a saturated or unsaturated (C₁-C₁₂)-alkyl group, a halogen-substituted (C₁-C₁₂)-alkyl group, a hydroxyl-(C₁-C₁₂)-alkyl group, a (C₁-C₆)-alkoxy-(C₁-C₁₂)-alkyl group, an amino-(C₁-C₁₂)-alkyl group, a (C₁-C₆)-alkylamino-(C₁-C₁₂)-alkyl group, a di(C₁-C₆)-alkylamino-(C₁-C₁₂)-alkyl group, a cyano-(C₁-C₁₂)-alkyl group, a substituted or unsubstituted phenyl group, a substituted or unsubstituted benzyl group or a substituted or unsubstituted heteroaryl group;
**R5, R6, R7** and **R8** may be identical or different and, independently of one another, are hydrogen, a halogen atom, a saturated or unsaturated (C₁-C₁₂)-alkyl group, a hydroxy group, a (C₁-C₁₂)-alkoxy group, a nitro group, an amino group, a (C₁-C₁₂)-alkylamino group or a di(C₁-C₁₂)-alkylamino group; and
**A⁻** is an anion of an organic or inorganic acid; for the colouring of keratin fibres.

2. Use according to Claim 1, **characterized in that R1** is a saturated or unsaturated (C₁-C₁₂)-alkyl group and **R4** is hydrogen, a saturated or unsaturated (C₁-C₁₂)-alkyl group, a substituted or unsubstituted phenyl group.

3. Use according to Claim 2, **characterized in that R1** is a saturated (C₁-C₁₂)-alkyl group and **R4** is hydrogen, a saturated (C₁-C₁₂)-alkyl group or an unsubstituted phenyl group.

4. Use according to one of Claims 1 to 3, **characterized in that A⁻** is a chloride, bromide, iodide, hydrogensulphate, sulphate, toluenesulphonate, benzenesulphonate, monomethylsulphate, hexafluorophosphate, hexafluoroantimonate, tetrafluoroborate, tetraphenylborate, formate, acetate or propionate anion.

5. Use according to one of Claims 1 to 4, **characterized in that** the compound of the formula (I) is chosen from 3-methyl-2-[(1-methyl-2-phenyl-1H-indol-3-yl)azo]thiazolium chloride, 3-methyl-2-[(1-methyl-2-phenyl-1H-indol-3-yl)azo]thiazolium bromide, 3-methyl-2-[(1-methyl-2-phenyl-1H-indol-3-yl)azo]thiazolium monomethylsulphate, 3,4-dimethyl-2-[(1-methyl-2-phenyl-1H-indol-3-yl)azo]thiazolium chloride, 3,4-dimethyl-2-[(1-methyl-2-phenyl-1H-indol-3-yl)azo]thiazolium bromide, 3,4-dimethyl-2-[(1-methyl-2-phenyl-1H-indol-3-yl)azo]thiazolium monomethylsulphate, 3,5-dimethyl-2-[(1-methyl-2-phenyl-1H-indol-3-yl)azo]thiazolium chloride, 3,5-dimethyl-2-[(1-methyl-2-phenyl-1H-indol-3-yl)azo]thiazolium bromide, 3,5-dimethyl-2-[(1-methyl-2-phenyl-1H-indol-3-yl)azo]thiazolium monomethylsulphate, 3,4,5-trimethyl-2-[(1-methyl-2-phenyl-1H-indol-3-yl)azo]thiazolium chloride, 3,4,5-trimethyl-2-[(1-methyl-2-phenyl-1H-indol-3-yl)azo]thiazolium bromide, 3,4,5-trimethyl-2-[(1-methyl-2-phenyl-1H-indol-3-yl)azo]thiazolium monomethylsulphate, 5-bromo-3-methyl-2-[(1-methyl-2-phenyl-1H-indol-3-yl)azo]thiazolium chloride, 5-bromo-3-methyl-2-[(1-methyl-2-phenyl-1H-indol-3-yl)azo]thiazolium bromide, 5-bromo-3-methyl-2-[(1-methyl-2-phenyl-1H-indol-3-yl)azo]thiazolium monomethylsulphate, 5-methoxy-3-methyl-2-[(1-methyl-2-phenyl-1H-indol-3-yl)azo]thiazolium chloride, 5-methoxy-3-methyl-2-[(1-methyl-2-phenyl-1H-indol-3-yl)azo]thiazolium bromide, 5-methoxy-3-methyl-2-[(1-methyl-2-phenyl-1H-indol-3-yl)azo]thiazolium monomethylsulphate, 3-methyl-2-[(2-phenyl-1H-indol-3-yl)azo]thiazolium chloride, 3-methyl-2-[(2-phenyl-1H-indol-3-yl)azo]thiazolium bromide, 3-methyl-2-[(2-phenyl-1H-indol-3-yl)azo]thiazolium monomethylsulphate, 3,4-dimethyl-2-[(2-phenyl-1H-indol-3-yl)azo]thiazolium chloride, 3,4-dimethyl-2-[(2-phenyl-1H-indol-3-yl)azo]thiazolium bromide, 3,4-dimethyl-2-[(2-phenyl-1H-indol-3-yl)azo]thiazolium monomethylsulphate, 3,5-dimethyl-2-[(2-phenyl-1H-indol-3-yl)azo]thiazolium chloride, 3,5-dimethyl-2-[(2-phenyl-1H-indol-3-yl)azo]thiazolium bromide, 3,5-dimethyl-2-[(2-phenyl-1H-indol-3-yl)azo]thiazolium monomethylsulphate, 3,4,5-trimethyl-2-[(2-phenyl-1H-indol-3-yl)azo]thiazolium chloride, 3,4,5-trimethyl-2-[(2-phenyl-1H-indol-3-yl)azo]thiazolium bromide, 3,4,5-trimethyl-2-[(2-phenyl-1H-indol-3-yl)azo]thiazolium monomethylsulphate, 5-bromo-3-methyl-2-[(2-phenyl-1H-indol-3-yl)azo]thiazolium chloride, 5-bromo-3-methyl-2-[(2-phenyl-1H-indol-3-yl)azo]thiazolium bromide, 5-bromo-3-methyl-2-[(2-phenyl-1H-indol-3-yl)azo]thiazolium monomethylsulphate, 5-methoxy-3-methyl-2-[(1,2-dimethyl-1H-indol-3-yl)azo]thiazolium chloride, 5-methoxy-3-methyl-2-[(2-phenyl-1H-indol-3-yl)azo]thiazolium bromide, 5-methoxy-3-methyl-2-[(2-phenyl-1H-indol-3-yl)azo]thiazolium monomethylsulphate, 3-methyl-2-[(1,2-diphenyl-1H-indol-3-yl)azo]thiazolium chloride, 3-methyl-2-[(1,2-diphenyl-1H-indol-3-yl)azo]-thiazolium bromide, 3-methyl-2-[(1,2-diphenyl-1H-indol-3-yl)azo]thiazolium monomethylsulphate, 3,4-dimethyl-2-[(1,2-diphenyl-1H-indol-3-yl)azo]thiazolium chloride, 3,4-dimethyl-2-[(1,2-diphenyl-1H-indol-3-yl)azo]thiazolium bromide, 3,4-dimethyl-2-[(1,2-diphenyl-1H-indol-3-yl)azo]thiazolium monomethylsulphate, 3,5-dimethyl-2-[(1,2-diphenyl-1H-indol-3-yl)azo]thiazolium chloride, 3,5-dimethyl-2-[(1,2-diphenyl-1H-indol-3-yl)azo]thiazolium bromide, 3,5-dimethyl-2-[(1,2-diphenyl-1H-indol-3-yl)azo]thiazolium monomethylsulphate, 3,4,5-trimethyl-2-[(1,2-diphenyl-1H-indol-3-yl)azo]thiazolium chloride, 3,4,5-trimethyl-2-[(1,2-diphenyl-1H-indol-3-yl)azo]thiazolium bromide, 3,4,5-trimethyl-2-[(1,2-diphenyl-1H-indol-3-yl)azo]thiazolium monomethylsulphate, 5-bromo-3-methyl-2-[(1,2-diphenyl-1H-indol-3-yl)azo]thiazolium chloride, 5-bromo-3-methyl-2-[(1,2-diphenyl-1H-indol-3-yl)azo]thiazolium bromide, 5-bromo-3-methyl-2-[(1,2-diphenyl-1H-indol-3-yl)azo]thiazolium monomethylsulphate, 5-methoxy-3-methyl-2-[(1,2-diphenyl-1H-indol-3-yl)azo]thiazolium chloride, 5-methoxy-3-methyl-2-[(1,2-diphenyl-1H-indol-3-yl)azo]-thiazolium bromide and 5-methoxy-3-methyl-2-[(1,2-diphenyl-1H-indol-3-yl)azo]thiazolium monomethylsulphate.

6. Agent for the colouring of keratin fibres, **characterized in that** it comprises at least one indolylthiazolium azo dye of the general formula (I), and also at least one further direct dye, 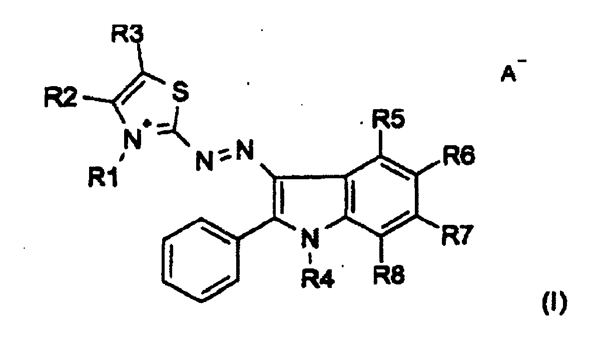 where
**R1** is a saturated or unsaturated (C₁-C₁₂)-alkyl group, a halogen-substituted (C₁-C₁₂)-alkyl group, a hydroxyl-(C₁-C₁₂) -alkyl group, a (C₁-C₆) -alkoxy- (C₁-C₁₂) -alkyl group, an amino-(C₁-C₁₂)-alkyl group, a (C₁-C₆)-alkylamino-(C₁-C₁₂)-alkyl group, a di(C₁-C₆)-alkylamino-(C₁-C₁₂)-alkyl group, a cyano-(C₁-C₁₂)-alkyl group, a substituted or unsubstituted phenyl group or a substituted or unsubstituted benzyl group,
**R2** and **R3** may be identical or different and, independently of one another, are hydrogen, a halogen atom, a saturated or unsaturated (C₁-C₁₂)-alkyl group, a (C₁-C₁₂)-alkoxy group, a nitro group, an amino group, a (C₁-C₁₂)-alkylamino group, a di(C₁-C₁₂)-alkylamino group, a (C₁-C₁₂)-hydroxyalkylamino group, a di(C₁-C₁₂)-hydroxyalkylamino group, a substituted or unsubstituted phenyl group or a substituted or unsubstituted heteroaryl group;
**R4** is hydrogen, a saturated or unsaturated (C₁-C₁₂)-alkyl group, a halogen-substituted (C₁-C₁₂)-alkyl group, a hydroxyl-(C₁-C₁₂)-alkyl group, a (C₁-C₆)-alkoxy-(C₁-C₁₂)-alkyl group, an amino-(C₁-C₁₂)-alkyl group, a (C₁-C₆)-alkylamino-(C₁-C₁₂)-alkyl group, a di(C₁-C₆)-alkylamino-(C₁-C₁₂)-alkyl group, a cyano-(C₁-C₁₂)-alkyl group, a substituted or unsubstituted phenyl group, a substituted or unsubstituted benzyl group or a substituted or unsubstituted heteroaryl group;
R5, R6, R7 and R8 may be identical or different and, independently of one another, are hydrogen, a halogen atom, a saturated or unsaturated (C₁-C₁₂)-alkyl group, a hydroxy group, a (C₁-C₁₂)-alkoxy group, a nitro group, an amino group, a (C₁-C₁₂)-alkylamino group or a di(C₁-C₁₂)-alkylamino group; and
A⁻ is an anion of an organic or inorganic acid.

7. Agent according to Claim 6, **characterized in that** the further direct dye is present in a total amount of from 0.01 to 4 per cent by weight.

8. Agent for the colouring of keratin fibres, **charcterized in that** it comprises at least one indolylthiazolium azo dye of the general formula (I), and at least one additional oxidation dye precursor and is mixed with an oxidizing agent before use, 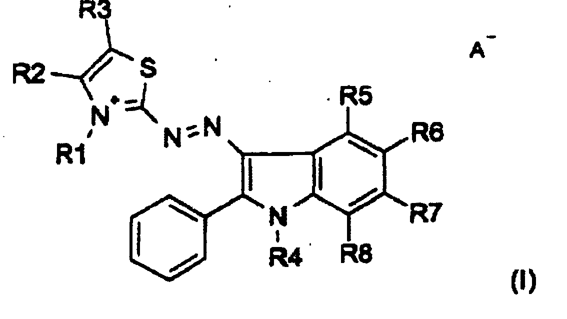 where
R1 is a saturated or unsaturated (C₁-C₁₂)-alkyl group, a halogen-substituted (C₁-C₁₂)-alkyl group, a hydroxyl-(C₁-C₁₂)-alkyl group, a (C₁-C₆)-alkoxy- (C₁-C₁₂)-alkyl group, an amino- (C₁-C₁₂)-alkyl group, a (C₁-C₆)-alkylamino- (C₁-C₁₂)-alkyl group, a di (C₁-C₆)-alkylamino-(C₁-C₁₂)-alkyl group, a cyano- (C₁-C₁₂)-alkyl group, a substituted or unsubstituted phenyl group or a substituted or unsubstituted benzyl group,
**R2** and **R3** may be identical or different and, independently of one another, are hydrogen, a halogen atom, a saturated or unsaturated (C₁-C₁₂)-alkyl group, a (C₁-C₁₂)-alkoxy group, a nitro group, an amino group, a (C₁-C₁₂)-alkylamino group, a di(C₁-C₁₂)-alkylamino group, a (C₁-C₁₂)-hydroxyalkylamino group, a di (C₁-C₁₂)-hydroxyalkylamino group, a substituted or unsubstituted phenyl group or a substituted or unsubstituted heteroaryl group;
**R4** is hydrogen, a saturated or unsaturated (C₁-C₁₂)-alkyl group, a halogen-substituted (C₁-C₁₂)-alkyl group, a hydroxyl- (C₁-C₁₂)-alkyl group, a (C₁-C₆)-alkoxy-(C₁-C₁₂)-alkyl group, an amino-(C₁-C₁₂)-alkyl group, a (C₁-C₆)-alkylamino-(C₁-C₁₂) -alkyl group, a di(C₁-C₆)-alkylamino-(C₁-C₁₂)-alkyl group, a cyano-(C₁-C₁₂)-alkyl group, a substituted or unsubstituted phenyl group, a substituted or unsubstituted benzyl group or a substituted or unsubstituted heteroaryl group;
**R5, R6, R7** and **R8** may be identical or different and, independently of one another, are hydrogen, a halogen atom, a saturated or unsaturated (C₁-C₁₂)-alkyl group, a hydroxy group, a (C₁-C₁₂)-alkoxy group, a nitro group, an amino group, a (C₁-C₁₂)-alkylamino group or a di(C₁-C₁₂)-alkylamino group; and
**A⁻** is an anion of an organic or inorganic acid.

9. Agent for the colouring of keratin fibres, **characterized in that** it comprises at least one indolythiazolium azo dye of the general formula (I), and also at least one natural polymer, synthetic polymer or modified polymer of natural origin customary for cosmetic compositions, and is present in the form of a tint fixer or colour fixer, 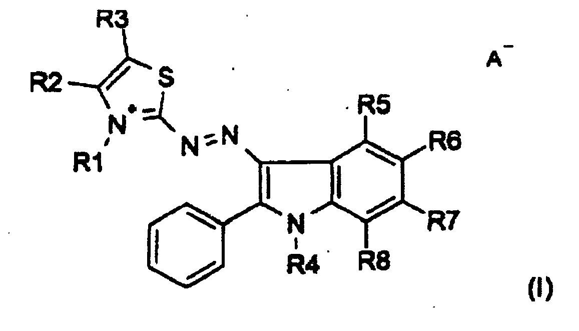 where
**R1** is a saturated (C₁-C₁₂)-alkyl group, a halogen-substituted (C₁-C₁₂)-alkyl group, a hydroxyl-(C₁-C₁₂)-alkyl group, a (C₁-C₆)-alkoxy-(C₁-C₁₂)-alkyl group, an amino- (C₁-C₁₂)-alkyl group, a (C₁-C₆)-alkylamino-(C₁-C₁₂)-alkyl group, a di(C₁-C₆)-alkylamino-(C₁-C₁₂)-alkyl group, a cyano-(C₁-C₁₂)-alkyl group, a substituted or unsubstituted phenyl group or a substituted or unsubstituted benzyl group,
**R2** and **R3** may be identical or different and, independently of one another, are hydrogen, a halogen atom, a saturated or unsaturated (C₁-C₁₂)-alkyl group, a (C₁-C₁₂)-alkoxy group, a nitro group, an amino group, a (C₁-C₁₂)-alkylamino group, a di(C₁-C₁₂)-alkylamino group, a (C₁-C₁₂) -hydroxyalkylamino group, a di (C₁-C₁₂)-hydroxyalkylamino group, a substituted or unsubstituted phenyl group or a substituted or unsubstituted heteroaryl group;
**R4** is hydrogen, a saturated or unsaturated (C₁-C₁₂)-alkyl group, a halogen-substituted (C₁-C₁₂)-alkyl group, a hydroxyl-(C₁-C₁₂)-alkyl group, a (C₁-C₆)-alkoxy-(C₁-C₁₂)-alkyl group, an amino-(C₁-C₁₂)-alkyl group, a (C₁-C₆)-alkylamino-(C₁-C₁₂) -alkyl group, a di(C₁-C₆)-alkylamino-(C₁-C₁₂)-alkyl group, a cyano-(C₁-C₁₂)-alkyl group, a substituted or unsubstituted phenyl group, a substituted or unsubstituted benzyl group or a substituted or unsubstituted heteroaryl group;
**R5, R6, R7** and **R8** may be identical or different and, independently of one another, are hydrogen, a halogen atom, a saturated or unsaturated (C₁-C₁₂)-alkyl group, a hydroxy group, a (C₁-C₁₂)-alkoxy group, a nitro group, an amino group, a (C₁-C₁₂) -alkylamino group or a di(C₁-C₁₂)-alkylamino group; and
**A**⁻ is an anion of an organic or inorganic acid.

10. Agent according to one of Claims 6 to 8, **characterized in that R1** is a saturated or unsaturated (C₁-C₁₂)-alkyl group and **R4** is hydrogen, a saturated or unsaturated (C₁-C₁₂)-alkyl group, a substituted or unsubstituted phenyl group.

11. Agent according to one of Claims 6 to 10, **characterized in that R1** is a saturated (C₁-C₁₂)-alkyl group and **R4** is hydrogen, a saturated (C₁-C₁₂)-alkyl group or an unsubstituted phenyl group.

12. Agent according to one of Claims 6 to 11, **characterized in that A⁻** is a chloride, bromide, iodide, hydrogensulphate, sulphate, toluenesulphonate, benzenesulphonate, monomethylsulphate, hexafluorophosphate, hexafluoroantimonate, tetrafluoroborate, tetraphenylborate, formate, acetate or propionate anion.

13. Agent according to one of Claims 6 to 12, **characterized in that** the compound of the formula (I) is chosen from 3-methyl-2-[(1-methyl-2-phenyl-1H-indol-3-yl)azo]thiazolium chloride, 3-methyl-2-[(1-methyl-2-phenyl-1H-indol-3-yl)azo]thiazolium bromide, 3-methyl-2-[(1-methyl-2-phenyl-1H-indol-3-yl)azo]thiazolium monomethylsulphate, 3,4-dimethyl-2-[(1-methyl-2-phenyl-1H-indol-3-yl)azo]thiazolium chloride, 3,4-dimethyl-2-[(1-methyl-2-phenyl-1H-indol-3-yl)azo]thiazolium bromide, 3,4-dimethyl-2-[(1-methyl-2-phenyl-1H-indol-3-yl)azo]thiazolium monomethylsulphate, 3,5-dimethyl-2-[(1-methyl-2-phenyl-1H-indol-3-yl)azo]thiazolium chloride, 3,5-dimethyl-2-[(1-methyl-2-phenyl-1H-indol-3-yl)azo]thiazolium bromide, 3,5-dimethyl-2-[(1-methyl-2-phenyl-1H-indol-3-yl)azo]thiazolium monomethylsulphate, 3,4,5-trimethyl-2-[(1-methyl-2-phenyl-1H-indol-3-yl)azo]thiazolium chloride, 3,4,5-trimethyl-2-[(1-methyl-2-phenyl-1H-indol-3-yl)azo]thiazolium bromide, 3,4,5-trimethyl-2-[(1-methyl-2-phenyl-1H-indol-3-yl)azo]thiazolium monomethylsulphate, 5-bromo-3-methyl-2-[(1-methyl-2-phenyl-1H-indol-3-yl)azo]thiazolium chloride, 5-bromo-3-methyl-2-[(1-methyl-2-phenyl-1H-indol-3-yl)azo]thiazolium bromide, 5-bromo-3-methyl-2-[(1-methyl-2-phenyl-1H-indol-3-yl)azo]thiazolium monomethylsulphate, 5-methoxy-3-methyl-2-[(1-methyl-2-phenyl-1H-indol-3-yl)azo]thiazolium chloride, 5-methoxy-3-methyl-2-[(1-methyl-2-phenyl-1H-indol-3-yl)azo]thiazolium bromide, 5-methoxy-3-methyl-2-[(1-methyl-2-phenyl-1H-indol-3-yl)azo]thiazolium monomethylsulphate, 3-methyl-2-[(2-phenyl-1H-indol-3-yl)azo]thiazolium chloride, 3-methyl-2-[(2-phenyl-1H-indol-3-yl)azo]thiazolium bromide, 3-methyl-2-[(2-phenyl-1H-indol-3-yl)azo]thiazolium monomethylsulphate, 3,4-dimethyl-2-[(2-phenyl-1H-indol-3-yl)azo]thiazolium chloride, 3,4-dimethyl-2-[(2-phenyl-1H-indol-3-yl)azo]thiazolium bromide, 3,4-dimethyl-2-[(2-phenyl-1H-indol-3-yl)azo]thiazolium monomethylsulphate, 3,5-dimethyl-2-[(2-phenyl-1H-indol-3-yl)azo]thiazolium chloride, 3,5-dimethyl-2-[(2-phenyl-1H-indol-3-yl)azo]thiazolium bromide, 3,5-dimethyl-2-[(2-phenyl-1H-indol-3-yl)azo]thiazolium monomethylsulphate, 3,4,5-trimethyl-2-[(2-phenyl-1H-indol-3-yl)azo]thiazolium chloride, 3,4,5-trimethyl-2-[(2-phenyl-1H-indol-3-yl)azo]thiazolium bromide, 3,4,5-trimethyl-2-[(2-phenyl-1H-indol-3-yl)azo]thiazolium monomethylsulphate, 5-bromo-3-methyl-2-[(2-phenyl-1H-indol-3-yl)azo]thiazolium chloride, 5-bromo-3-methyl-2-[(2-phenyl-1H-indol-3-yl)azo]thiazolium bromide, 5-bromo-3-methyl-2-[(2-phenyl-1H-indol-3-yl)azo]thiazolium monomethylsulphate, 5-methoxy-3-methyl-2-[(1,2-dimethyl-1H-indol-3-yl)azo]thiazolium chloride, 5-methoxy-3-methyl-2-[(2-phenyl-1H-indol-3-yl)azo]thiazolium bromide, 5-methoxy-3-methyl-2-[(2-phenyl-1H-indol-3-yl)azo]thiazolium monomethylsulphate, 3-methyl-2-[(1,2-diphenyl-1H-indol-3-yl)azo]thiazolium chloride, 3-methyl-2-[(1,2-diphenyl-1H-indol-3-yl)azo]-thiazolium bromide, 3-methyl-2-[(1,2-diphenyl-1H-indol-3-yl)azo]thiazolium monomethylsulphate, 3,4-dimethyl-2-[(1,2-diphenyl-1H-indol-3-yl)azo]thiazolium chloride, 3,4-dimethyl-2-[(1,2-diphenyl-1H-indol-3-yl)azo]thiazolium bromide, 3,4-dimethyl-2-[(1,2-diphenyl-1H-indol-3-yl)azo]thiazolium monomethylsulphate, 3,5-dimethyl-2-[(1,2-diphenyl-1H-indol-3-yl)azo]thiazolium chloride, 3,5-dimethyl-2-[(1,2-diphenyl-1H-indol-3-yl)azo]thiazolium bromide, 3,5-dimethyl-2-[(1,2-diphenyl-1H-indol-3-yl)azo]thiazolium monomethylsulphate, 3,4,5-trimethyl-2-[(1,2-diphenyl-1H-indol-3-yl)azo]thiazolium chloride, 3,4,5-trimethyl-2-[(1,2-diphenyl-1H-indol-3-yl)azo]thiazolium bromide, 3,4,5-trimethyl-2-[(1,2-diphenyl-1H-indol-3-yl)azo]thiazolium monomethylsulphate, 5-bromo-3-methyl-2-[(1,2-diphenyl-1H-indol-3-yl)azo]thiazolium chloride, 5-bromo-3-methyl-2-[(1,2-diphenyl-1H-indol-3-yl)azo]thiazolium bromide, 5-bromo-3-methyl-2-[(1,2-diphenyl-1H-indol-3-yl)azo]thiazolium monomethylsulphate, 5-methoxy-3-methyl-2-[(1,2-diphenyl-1H-indol-3-yl)azo]thiazolium chloride, 5-methoxy-3-methyl-2-[(1,2-diphenyl-1H-indol-3-yl)azo]-thiazolium bromide and 5-methoxy-3-methyl-2-[(1,2-diphenyl-1H-indol-3-yl)azo]thiazolium monomethylsulphate.

14. Agent according to one of Claims 6 to 13, **characterized in that** the compound of formula (I) is present in an amount of 0.01 to 10 per cent by weight.

15. Agent according to one of Claims 6 to 14, **characterized in that** it is a hair colorant.

## Revendications

1. Utilisation d'une composition qui contient au moins un colorant azoïque indolylthidzolium de formule générale (I), 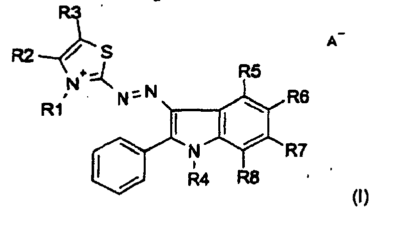 formule dans laquelle
**R1** représente un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy(C₁-C₆)-alkyle(C₁-C₁₂), un groupe aminoalkyle en C₁-C₁₂, un groupe alkyl(C₁-C₆)aminoalkyle(C₁-C₁₂), un groupe dialkyl(C₁-C₆)aminoalkyle(C₁-C₁₂), un groupe cyanoalkyle(C₁-C₁₂), un groupe phényle substitué ou non substitué ou un groupe benzyle substitué ou non substitué ;
**R2** et **R3** peuvent être identiques ou différents et représentent, indépendamment l'un de l'autre, un atome d'hydrogène, un atome d'halogène, un groupe alkyle en C₁-C₁₂ saturé ou insaturé, un groupe alcoxy en C₁-C₁₂, un groupe nitro, un groupe amino, un groupe alkyl(C₁-C₁₂)amino, un groupe dialkyl(C₁-C₁₂)amino, un groupe hydroxyalkyl(C₁-C₁₂) amino, un groupe dihydroxyalkyl(C₁-C₁₂)amino, un groupe phényle substitué ou non substitué ou un groupe hétéroaryle substitué ou non substitué ;
**R4** représente un atome d'hydrogène, un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy(C₁-C₆)-alkyle(C₁-C₁₂), un groupe aminoalkyle en C₁-C₁₂, un groupe alkyl(C₁-C₆)amino-alkyle(C₁-C₁₂), un groupe dialkyl(C₁-C₆)amino-alkyle(C₁-C₁₂), un groupe cyanoalkyle(C₁-C₁₂), un groupe phényle substitué ou non substitué, un groupe benzyle substitué ou non substitué ou un groupe hétéroaryle substitué ou non substitué ;
**R5, R6, R7** et **R9** peuvent être identiques ou différents et représentent, indépendamment les uns des autres, un atome d'hydrogène, un atome d'halogène, un groupe alkyle en C₁-C₁₂ saturé ou insaturé, un groupe hydroxy, un groupe alcoxy en C₁-C₁₂, un groupe nitro, un groupe amino, un groupe alkyl(C₁-C₁₂)amino ou un groupe dialkyl(C₁-C₁₂)amino ; et
**A⁻** est un anion d'un acide organique ou minéral ; destinée à colorer les fibres de kératine.

2. Utilisation selon la revendication 1, **caractérisée en ce que R1** est un groupe alkyle en C₁-C₁₂ saturé ou insaturé et **R4** est un atome d'hydrogène, un groupe alkyle en C₁-C₁₂ saturé ou insaturé, un groupe phényle substitué ou non substitué.

3. Utilisation selon la revendication 2, **caractérisée en ce que R1** est un groupe alkyle en C₁-C₁₂ saturé et **R4** est un atome d'hydrogène, un groupe alkyle en C₁-C₁₂ saturé ou un groupe phényle.

4. Utilisation selon l'une quelconque des revendications 1 à 3, **caractérisée en ce que A⁻** est un anion chlorure, bromure, iodure, hydrogénosulfate, sulfate, toluènesulfonate, benzènesulfonate, monométhylsulfate, hexafluorophosphate, hexafluoroantimonate, tétrafluoroborate, tétraphénylborate, formiate, acétate ou propionate.

5. Utilisation selon l'une quelconque des revendications 1 à 4, **caractérisée en ce que** le composé de formule (I) est choisi parmi le chlorure de 3-méthyl-2-[(1-méthyl-2-phényl-1H-indol-3-yl)azo]-thiazolium, le bromure de 3-méthyl-2-[(1-méthyl-2-phényl-1H-indol-3-yl)azo]-thiazolium, le monométhylsulfate de 3-méthyl-2-[(1-méthyl-2-phényl-1H-indol-3-yl)azo]-thiazolium, le chlorure de 3,4-diméthyl-2-[(1-méthyl-2-phényl-1H-indol-3-yl)azo]-thiazolium, le bromure de 3,4-diméthyl-2-[(1-méthyl-2-phényl-1H-indol-3-yl)azo]-thiazolium, le monométhylsulfate de 3,4-diméthyl-2-[(1-méthyl-2-phényl-1H-indol-3-yl)azo]-thiazolium, le chlorure de 3,5-diméthyl-2-[(1-méthyl-2-phényl-1H-indol-3-yl)azo]-thiazolium, le bromure de 3,5-diméthyl-2-[(1-méthyl-2-phényl-1H-indol-3-yl)azo]-thiazolium, le monométhylsulfate de 3,5-diméthyl-2-[(1-méthyl-2-phényl-1H-indol-3-yl)azo]-thiazolium, le chlorure de 3,4,5-triméthyl-2-[(1-méthyl-2-phényl-1H-indol-3-yl)azo]-thiazolium, le bromure de 3,4,5-triméthyl-2-[(1-méthyl-2-phényl-1H-indol-3-yl)azo]-thiazolium, le monométhylsulfate de 3,4,5-triméthyl-2-[(1-méthyl-2-phényl-1H-indol-3-yl)azo]-thiazolium, le chlorure de 5-bromo-3-méthyl-2-[(1-méthyl-2-phényl-1H-indol-3-yl)azo]-thiazolium, le bromure de 5-bromo-3-méthyl-2-[(1-méthyl-2-phényl-1H-indol-3-yl)azo]-thiazolium, le monométhylsulfate de 5-bromo-3-méthyl-2-[(1-méthyl-2-phényl-1H-indol-3-yl)azo]-thiazolium, le chlorure de 5-méthoxy-3-méthyl-2-[(1-méthyl-2-phényl-1H-indol-3-yl)azo]-thiazolium, le bromure de 5-méthoxy-3-méthyl-2-[(1-méthyl-2-phényl-1H-indol-3-yl)azo]-thiazolium, le monométhylsulfate de 5-méthoxy-3-méthyl-2-[(1-méthyl-2-phényl-1H-indol-3-yl)azo]-thiazolium, le chlorure de 3-méthyl-2-[(2-phényl-1H-indol-3-yl)azo]-thiazolium, le bromure de 3-méthyl-2-[(2-phényl-1H-indol-3-yl)azo]-thiazolium, le monométhylsulfate de 3-méthyl-2-[(2-phényl-1H-indol-3-yl)azo]-thiazolium, le chlorure de 3,4-diméthyl-2-[(2-phényl-1H-indol-3-yl)azo]-thiazolium, le bromure de 3,4-diméthyl-2-[(2-phényl-1H-indol-3-yl)azo]-thiazolium, le monométhylsulfate de 3,4-diméthyl-2-[(2-phényl-1H-indol-3-yl)azo]-thiazolium, le chlorure de 3,5-diméthyl-2-[(2-phényl-1H-indol-3-yl)azo]-thiazolium, le bromure de 3,5-diméthyl-2-[(2-phényl-1H-indol-3-yl)azo]-thiazolium, le monométhylsulfate de 3,5-diméthyl-2-[(2-phényl-1H-indol-3-yl)azo]-thiazolium, le chlorure de 3,4,5-triméthyl-2-[(2-phényl-1H-indol-3-yl)azo]-thiazolium, le bromure de 3,4,5-triméthyl-2-[(2-phényl-1H-indol-3-yl)azo]-thiazolium, le monométhylsulfate de 3,4,5-triméthyl-2-[(2-phényl-1H-indol-3-yl)azo]-thiazolium, le chlorure de 5-bromo-3-méthyl-2-[(2-phényl-1H-indol-3-yl)azo]-thiazolium, le bromure de 5-bromo-3-méthyl-2-[(2-phényl-1H-indol-3-yl)azo]-thiazolium, le monométhylsulfate de 5-bromo-3-méthyl-2-[(2-phényl-1H-indol-3-yl)azo]-thiazolium, le chlorure de 5-méthoxy-3-méthyl-2-[(2-phényl-1H-indol-3-yl)azo]-thiazolium, le bromure de 5-méthoxy-3-méthyl-2-[(2-phényl-1H-indol-3-yl)azo]-thiazolium, le monométhylsulfate de 5-méthoxy-3-méthyl-2-[(2-phényl-1H-indol-3-yl)azo]-thiazolium, le monométhylsulfate de 3-méthyl-2-[(1,2-diméthyl-1H-indol-3-yl)azo]-5-nitrothiazolium, le chlorure de 3-méthyl-2-[(1,2-diphényl-1H-indol-3-yl)azo]-thiazolium, le bromure de 3-méthyl-2-[(1,2-diphényl-1H-indol-3-yl)azo]-thiazolium, le monométhylsulfate de 3-méthyl-2-[(1,2-diphényl-1H-indol-3-yl)azo]thiazolium, le chlorure de 3,4-diméthyl-2-[(1,2-diphényl-1H-indol-3-yl)azo]-thiazolium, le bromure de 3,4-diméthyl-2-[(1,2-diphényl-1H-indol-3-yl)azo]-thiazolium, le monométhylsulfate de 3,4-diméthyl-2-[(1,2-diphényl-1H-indol-3-yl)azo]-thiazolium, le chlorure de 3,5-diméthyl-2-[(1,2-diphényl-1H-indol-3-yl)azo]-thiazolium, le bromure de 3,5-diméthyl-2-[(1,2-diphényl-1H-indol-3-yl)azo]-thiazolium, le monométhylsulfate de 3,5-diméthyl-2-[(1,2-diphényl-1H-indol-3-yl)azo]-thiazolium, le chlorure de 3,4,5-triméthyl-2-[(1,2-diphényl-1H-indol-3-yl)azo]-thiazolium, le bromure de 3,4,5-triméthyl-2-[(1,2-diphényl-1H-indol-3-yl)azo]-thiazolium, le monométhylsulfate de 3,4,5-triméthyl-2-[(1,2-diphényl-1H-indol-3-yl)azo]-thiazolium, le chlorure de 5-bromo-3-méthyl-2-[(1,2-diphényl-1H-indol-3-yl)azo]-thiazolium, le bromure de 5-bromo-3-méthyl-2-[(1,2-diphényl-1H-indol-3-yl)azo]-thiazolium, le monométhylsulfate de 5-bromo-3-méthyl-2-[(1,2-diphényl-1H-indol-3-yl)azo]-thiazolium, le chlorure de 5-méthoxy-3-méthyl-2-[(1,2-diphényl-1H-indol-3-yl)azo]-thiazolium, le bromure de 5-méthoxy-3-méthyl-2-[(1,2-diphényl-1H-indol-3-yl)azo]-thiazolium et le monométhylsulfate de 5-méthoxy-3-méthyl-2-[(1,2-diphényl-1H-indol-3-yl)azo]-thiazolium.

6. Composition destinée à colorer les fibres de kératine, **caractérisée en ce qu'**elle contient au moins un colorant azoïque indolylthiazolium de formule générale (I), en plus qu'au moins un colorant direct additionnel 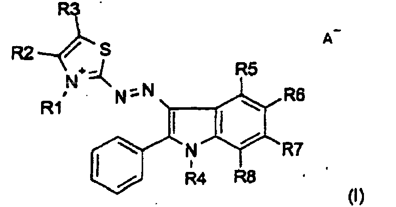 formule dans laquelle
**R1** représente un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy(C₁-C₆)-alkyle(C₁-C₁₂), un groupe aminoalkyle en C₁-C₁₂, un groupe alkyl(C₁-C₆)aminoalkyle(C₁-C₁₂), un groupe dialkyl(C₁-C₆)aminoalkyle(C₁-C₁₂), un groupe cyanoalkyle(C₁-C₁₂), un groupe phényle substitué ou non substitué ou un groupe benzyle substitué ou non substitué ;
**R2** et **R3** peuvent être identiques ou différents et représentent, indépendamment l'un de l'autre, un atome d'hydrogène, un atome d'halogène, un groupe alkyle en C₁-C₁₂ saturé ou insaturé, un groupe alcoxy en C₁-C₁₂, un groupe nitro, un groupe amino, un groupe alkyl(C₁-C₁₂)amino, un groupe dialkyl(C₁-C₁₂)amino, un groupe hydroxyalkyl(C₁-C₁₂) amino, un groupe dihydroxyalkyl(C₁-C₁₂)amino, un groupe phényle substitué ou non substitué ou un groupe hétéroaryle substitué ou non substitué ;
**R4** représente un atome d'hydrogène, un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy(C₁-C₆)-alkyle(C₁-C₁₂), un groupe aminoalkyle en C₁-C₁₂, un groupe alkyl(C₁-C₆)amino-alkyle(C₁-C₁₂), un groupe dialkyl(C₁-C₆)amino-alkyle(C₁-C₁₂), un groupe cyanoalkyle(C₁-C₁₂), un groupe phényle substitué ou non substitué, un groupe benzyle substitué ou non substitué ou un groupe hétéroaryle substitué ou non substitué ;
**R5, R6, R7** et **R9** peuvent être identiques ou différents et représentent, indépendamment les uns des autres, un atome d'hydrogène, un atome d'halogène, un groupe alkyle en C₁-C₁₂ saturé ou insaturé, un groupe hydroxy, un groupe alcoxy en C₁-C₁₂, un groupe nitro, un groupe amino, un groupe alkyl(C₁-C₁₂) amino ou un groupe dialkyl(C₁-C₁₂)amino ; et
**A⁻** est un anion d'un acide organique ou minéral.

7. Composition selon la revendication 6, **caractérisée en ce que** le colorant direct additionnel est contenu en une quantité totale de 0,01 à 4 % en poids.

8. Composition destinée à colorer les fibres de kératine, **caracterisée en ce qu**'elle contient au moins un colorant azoïque indolylthiazolium de formule générale (I), en plus qu'au moins un précurseur de colorants d'oxydation et qu'on la mélange avec une composition d'oxydation avant la utilisation 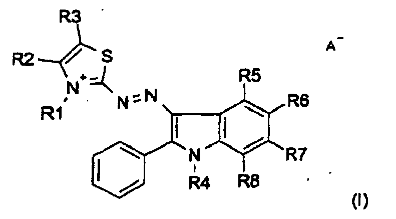 formule dans laquelle
**R1** représente un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy(C₁-C₆)-alkyle(C₁-C₁₂), un groupe aminoalkyle en C₁-C₁₂, un groupe alkyl(C₁-C₆)aminoalkyle(C₁-C₁₂), un groupe dialkyl (C₁-C₆) aminoalkyle(C₁-C₁₂), un groupe cyanoalkyle(C₁-C₁₂), un groupe phényle substitué ou non substitué ou un groupe benzyle substitué ou non substitué ;
**R2** et **R3** peuvent être identiques ou différents et représentent, indépendamment l'un de l'autre, un atome d'hydrogène, un atome d'halogène, un groupe alkyle en C₁-C₁₂ saturé ou insaturé, un groupe alcoxy en C₁-C₁₂, un groupe nitro, un groupe amino, un groupe alkyl(C₁-C₁₂)amino, un groupe dialkyl(C₁-C₁₂)amino, un groupe hydroxyalkyl(C₁-C₁₂)amino, un groupe dihydroxyalkyl(C₁-C₁₂)amino, un groupe phényle substitué ou non substitué ou un groupe hétéroaryle substitué ou non substitué ;
**R4** représente un atome d'hydrogène, un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy(C₁-C₆)-alkyle(C₁-C₁₂), un groupe aminoalkyle en C₁-C₁₂, un groupe alkyl(C₁-C₆)amino-alkyle(C₁-C₁₂), un groupe dialkyl(C₁-C₆)amino-alkyle(C₁-C₁₂), un groupe cyanoalkyle(C₁-C₁₂), un groupe phényle substitué ou non substitué, un groupe benzyle substitué ou non substitué ou un groupe hétéroaryle substitué ou non substitué ;
**R5, R6, R7** et **R9** peuvent être identiques ou différents et représentent, indépendamment les uns des autres, un atome d'hydrogène, un atome d'halogène, un groupe alkyle en C₁-C₁₂ saturé ou insaturé, un groupe hydroxy, un groupe alcoxy en C₁-C₁₂, un groupe nitro, un groupe amino, un groupe alkyl(C₁-C₁₂)amino ou un groupe dialkyl(C₁-C₁₂)amino ; et
**A⁻** est un anion d'un acide organique ou minéral.

9. Composition destinée à colorer les fibres de kératine, **caracterisée en ce qu**'elle contient au moins un colorant azoïque indolylthiazolium de formule générale (I), en plus qu'au moins un polymère naturel, polymère synthétique ou polymère modifié d'origine naturelle qu'on utilise de convention pour les compositions cosmétiques, et se trouve sous forme de fixatif de ton ou fixatif de color 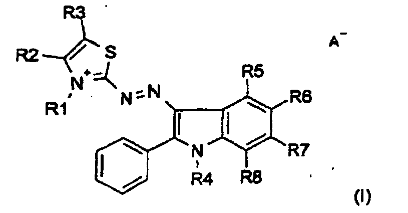 formule dans laquelle
**R1** représente un groupe alkyle en C₁-C₁₂ saturé, un groupe alkyle en C₁-C₁₂ substitué par un atome d'halogène, un groupe hydroxyalkyle en C₁-C₁₂, un groupe alcoxy(C₁-C₆)-alkyle(C₁-C₁₂), un groupe aminoalkyle en C₁-C₁₂, un groupe alkyl(C₁-C₆)amino-alkyle(C₁-C₁₂), un groupe dialkyl(C₁-C₆)amino-alkyle(C₁-C₁₂), un groupe cyanoalkyle(C₁-C₁₂), un groupe phényle substitué ou non substitué ou un groupe benzyle substitué ou non substitué ;
**R2** et **R3** peuvent être identiques ou différents et représentent, indépendamment l'un de l'autre, un atome d'hydrogène, un atome d'halogène, un groupe alkyle en C₁-C₁₂ saturé ou insaturé, un groupe alcoxy en C₁-C₁₂, un groupe nitro, un groupe amino, un groupe alkyl(C₁-C₁₂)amino, un groupe dialkyl(C₁-C₁₂)amino, un groupe hydroxyalkyl(C₁-C₁₂) amino, un groupe dihydroxyalkyl(C₁-C₁₂)amino, un groupe phényle substitué ou non substitué ou un groupe hétéroaryle substitué ou non substitué ;
**R4** représente un atome d'hydrogène, un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy(C₁-C₆)-alkyle(C₁-C₁₂), un groupe aminoalkyle en C₁-C₁₂, un groupe alkyl(C₁-C₆) amino-alkyle(C₁-C₁₂), un groupe dialkyl (C₁-C₆) amino-alkyle(C₁-C₁₂), un groupe cyanoalkyle(C₁-C₁₂), un groupe phényle substitué ou non substitué, un groupe benzyle substitué ou non substitué ou un groupe hétéroaryle substitué ou non substitué ;
**R5, R6, R7** et **R9** peuvent être identiques ou différents et représentent, indépendamment les uns des autres, un atome d'hydrogène, un atome d'halogène, un groupe alkyle en C₁-C₁₂ saturé ou insaturé, un groupe hydroxy, un groupe alcoxy en C₁-C₁₂, un groupe nitro, un groupe amino, un groupe alkyl(C₁-C₁₂)amino ou un groupe dialkyl(C₁-C₁₂)amino ; et
**A⁻** est un anion d'un acide organique ou minéral.

10. Composition selon l'une quelconque des revendications 6 à 8, **caractérisée en ce que R1** est un groupe alkyle en C₁-C₁₂ saturé ou insaturé et **R4** est un atome d'hydrogène, un groupe alkyle en C₁-C₁₂ saturé ou insaturé, un groupe phényle substitué ou non substitué.

11. Composition selon l'une quelconque des revendications 6 à 10, **caractérisée en ce que R1** est un groupe alkyle en C₁-C₁₂ saturé et **R4** est un atome d'hydrogène, un groupe alkyle en C₁-C₁₂ saturé ou un groupe phényle.

12. Composition selon l'une quelconque des revendications 6 à 11, **caractérisée en ce que A⁻** est un anion chlorure, bromure, iodure, hydrogénosulfate, sulfate, toluènesulfonate, benzènesulfonate, monométhylsulfate, hexafluorophosphate, hexafluoroantimonate, tétrafluoroborate, tétraphénylborate, formiate, acétate ou propionate.

13. Composition selon l'une quelconque des revendications 6 à 12, **caractérisée en ce que** le composé de formule (I) est choisi parmi le chlorure de 3-méthyl-2-[(1-méthyl-2-phényl-1H-indol-3-yl)azo]-thiazolium, le bromure de 3-méthyl-2-[(1-méthyl-2-phényl-1H-indol-3-yl)azo]-thiazolium, le monométhylsulfate de 3-méthyl-2-[(1-méthyl-2-phényl-1H-indol-3-yl)azo]-thiazolium, le chlorure de 3,4-diméthyl-2-[(1-méthyl-2-phényl-1H-indol-3-yl)azo]-thiazolium, le bromure de 3,4-diméthyl-2-[(1-méthyl-2-phényl-1H-indol-3-yl)azo]-thiazolium, le monométhylsulfate de 3,4-diméthyl-2-[(1-méthyl-2-phényl-1H-indol-3-yl)azo]-thiazolium, le chlorure de 3,5-diméthyl-2-[(1-méthyl-2-phényl-1H-indol-3-yl)azo]-thiazolium, le bromure de 3,5-diméthyl-2-[(1-méthyl-2-phényl-1H-indol-3-yl)azo]-thiazolium, le monométhylsulfate de 3,5-diméthyl-2-[(1-méthyl-2-phényl-1H-indol-3-yl)azo]-thiazolium, le chlorure de 3,4,5-triméthyl-2-[(1-méthyl-2-phényl-1H-indol-3-yl)azo]-thiazolium, le bromure de 3,4,5-triméthyl-2-[(1-méthyl-2-phényl-1H-indol-3-yl)azo]-thiazolium, le monométhylsulfate de 3,4,5-triméthyl-2-[(1-méthyl-2-phényl-1H-indol-3-yl)azo]-thiazolium, le chlorure de 5-bromo-3-méthyl-2-[(1-méthyl-2-phényl-1H-indol-3-yl)azo]-thiazolium, le bromure de 5-bromo-3-méthyl-2-[(1-méthyl-2-phényl-1H-indol-3-yl)azo]-thiazolium, le monométhylsulfate de 5-bromo-3-méthyl-2-[(1-méthyl-2-phényl-1H-indol-3-yl)azo]-thiazolium, le chlorure de 5-méthoxy-3-méthyl-2-[(1-méthyl-2-phényl-1H-indol-3-yl)azo]-thiazolium, le bromure de 5-méthoxy-3-méthyl-2-[(1-méthyl-2-phényl-1H-indol-3-yl)azo]-thiazolium, le monométhylsulfate de 5-méthoxy-3-méthyl-2-[(1-méthyl-2-phényl-1H-indol-3-yl)azo]-thiazolium, le chlorure de 3-méthyl-2-[(2-phényl-1H-indol-3-yl)azo]-thiazolium, le bromure de 3-méthyl-2-[(2-phényl-1H-indol-3-yl)azo]-thiazolium, le monométhylsulfate de 3-méthyl-2-[(2-phényl-1H-indol-3-yl)azo]-thiazolium, le chlorure de 3,4-diméthyl-2-[(2-phényl-1H-indol-3-yl)azo]-thiazolium, le bromure de 3,4-diméthyl-2-[(2-phényl-1H-indol-3-yl)azo]-thiazolium, le monométhylsulfate de 3,4-diméthyl-2-[(2-phényl-1H-indol-3-yl)azo]-thiazolium, le chlorure de 3,5-diméthyl-2-[(2-phényl-1H-indol-3-yl)azo]-thiazolium, le bromure de 3,5-diméthyl-2-[(2-phényl-1H-indol-3-yl)azo]-thiazolium, le monométhylsulfate de 3,5-diméthyl-2-[(2-phényl-1H-indol-3-yl)azo]-thiazolium, le chlorure de 3,4,5-triméthyl-2-[(2-phényl-1H-indol-3-yl)azo]-thiazolium, le bromure de 3,4,5-triméthyl-2-[(2-phényl-1H-indol-3-yl)azo]-thiazolium, le monométhylsulfate de 3,4,5-triméthyl-2-[(2-phényl-1H-indol-3-yl)azo]-thiazolium, le chlorure de 5-bromo-3-méthyl-2-[(2-phényl-1H-indol-3-yl)azo]-thiazolium, le bromure de 5-bromo-3-méthyl-2-[(2-phényl-1H-indol-3-yl)azo]-thiazolium, le monométhylsulfate de 5-bromo-3-méthyl-2-[(2-phényl-1H-indol-3-yl)azo]-thiazolium, le chlorure de 5-méthoxy-3-méthyl-2-[(2-phényl-1H-indol-3-yl)azo]-thiazolium, le bromure de 5-méthoxy-3-méthyl-2-[(2-phényl-1H-indol-3-yl)azo]-thiazolium, le monométhylsulfate de 5-méthoxy-3-méthyl-2-[(2-phényl-1H-indol-3-yl)azo]-thiazolium, le monométhylsulfate de 3-méthyl-2-[(1,2-diméthyl-1H-indol-3-yl)azo]-5-nitrothiazolium, le chlorure de 3-méthyl-2-[(1,2-diphényl-1H-indol-3-yl)azo]-thiazolium, le bromure de 3-méthyl-2-[(1,2-diphényl-1H-indol-3-yl)azo]-thiazolium, le monométhylsulfate de 3-méthyl-2-[(1,2-diphényl-1H-indol-3-yl)azo]thiazolium, le chlorure de 3,4-diméthyl-2-[(1,2-diphényl-1H-indol-3-yl)azo]-thiazolium, le bromure de 3,4-diméthyl-2-[(1,2-diphényl-1H-indol-3-yl)azo]-thiazolium, le monométhylsulfate de 3,4-diméthyl-2-[(1,2-diphényl-1H-indol-3-yl)azo]-thiazolium, le chlorure de 3,5-diméthyl-2-[(1,2-diphényl-1H-indol-3-yl)azo]-thiazolium, le bromure de 3,5-diméthyl-2-[(1,2-diphényl-1H-indol-3-yl)azo]-thiazolium, le monométhylsulfate de 3,5-diméthyl-2-[(1,2-diphényl-1H-indol-3-yl)azo]-thiazolium, le chlorure de 3,4,5-triméthyl-2-[(1,2-diphényl-1H-indol-3-yl)azo]-thiazolium, le bromure de 3,4,5-triméthyl-2-[(1,2-diphényl-1H-indol-3-yl)azo]-thiazolium, le monométhylsulfate de 3,4,5-triméthyl-2-[(1,2-diphényl-1H-indol-3-yl)azo]-thiazolium, le chlorure de 5-bromo-3-méthyl-2-[(1,2-diphényl-1H-indol-3-yl)azo]-thiazolium, le bromure de 5-bromo-3-méthyl-2-[(1,2-diphényl-1H-indol-3-yl)azo]-thiazolium, le monométhylsulfate de 5-bromo-3-méthyl-2-[(1,2-diphényl-1H-indol-3-yl)azo]-thiazolium, le chlorure de 5-méthoxy-3-méthyl-2-[(1,2-diphényl-1H-indol-3-yl)azo]-thiazolium, le bromure de 5-méthoxy-3-méthyl-2-[(1,2-diphényl-1H-indol-3-yl)azo]-thiazolium et le monométhylsulfate de 5-méthoxy-3-méthyl-2-[(1,2-diphényl-1H-indol-3-yl)azo]-thiazolium.

14. Composition selon l'une quelconque des revendications 6 à 13, **caractérisée en ce que** le composé de formule (I) est contenu en une quantité de 0,01 à 10 % en poids.

15. Composition selon l'une quelconque des revendications 6 à 14, **caractérisée en ce qu'**il s'agit d'une composition de teinture pour cheveux.
